# EUROPÄISCHE PATENTSCHRIFT

(11) **EP 1 776 451 B1**
(45) Veröffentlichungstag und Bekanntmachung des Hinweises auf die Patenterteilung: **14.09.2011**
(21) Anmeldenummer: 05757370.1
(22) Anmeldetag: 05.07.2005
(51) Int. Cl.: C12N 1/21, C12N 15/52, C12P 13/04, C12P 13/08

(54) **VERFAHREN ZUR HERSTELLUNG VON L-AMINOSÄUREN UNTER VERWENDUNG VON STÄMMEN DER FAMILIE ENTEROBACTERIACEAE**
METHOD FOR THE PRODUCTION OF L-AMINO ACIDS USING STRAINS FROM THE ENTEROBACTERACEAE FAMILY
PROCEDE DE PRODUCTION D'AMINOACIDES L AU MOYEN DE SOUCHES DE LA FAMILLE DES ENTEROBACTERIES

(30) Priorität: 03.08.2004 DE 102004037572
(43) Veröffentlichungstag der Anmeldung: 25.04.2007
(73) Patentinhaber: Evonik Degussa GmbH, 45128 Essen (DE)
(72) Erfinder: RIEPING, Mechthild, 33619 Bielefeld (DE)
(86) Internationale Anmeldenummer: PCT/EP2005/007212
(87) Internationale Veröffentlichungsnummer: WO 2006/015669

(56) Entgegenhaltungen:
- EP-A- 1 382 685
- WO-A-03/004670
- WO-A-03/008605
- WO-A-03/076635
- DE-A1- 10 102 823
- US-A- 4 278 765
- GHOSH ROBIN ET AL: "Overexpression of outer membrane porins in E. coli using pBluescript-derived vectors" GENE EXPRESSION, Bd. 7, Nr. 3, 1998, Seiten 149-161, XP009059445 ISSN: 1052-2166
- NEWTON S M C ET AL: "Studies of the anaerobically induced promoter pnirB and the improved expression of bacterial antigens" RESEARCH IN MICROBIOLOGY, ELSEVIER, AMSTERDAM, NL, Bd. 146, Nr. 3, 1995, Seiten 193-202, XP002328484 ISSN: 0923-2508
- KRUSE D ET AL: "Influence of threonine exporters on threonine production in Escherichia coli." APPLIED MICROBIOLOGY AND BIOTECHNOLOGY. JUL 2002, Bd. 59, Nr. 2-3, Juli 2002 (2002-07), Seiten 205-210, XP009059146 ISSN: 0175-7598
- CLEMENT J M ET AL: "GENE SEQUENCE OF THE LAMBDA RECEPTOR AN OUTER MEMBRANE PROTEIN OF ESCHERICHIA-COLI K-12" CELL, Bd. 27, Nr. 3 PART 2, 1981, Seiten 507-514, XP002360993 ISSN: 0092-8674
- WEI J ET AL: "Complete genome sequence and comparative genomics of Shigella flexneri serotype 2a strain 2457T." INFECTION AND IMMUNITY, Bd. 71, Nr. 5, Mai 2003 (2003-05), Seiten 2775-2786, XP002360994 ISSN: 0019-9567
- FRANCOZ E ET AL: "The maltoporin of Salmonella typhimurium: Sequence and folding model" RESEARCH IN MICROBIOLOGY 1990 FRANCE, Bd. 141, Nr. 9, 1990, Seiten 1039-1059, XP002360995 ISSN: 0923-2508

### SEQUENZPROTOKOLL

<110> Degussa AG
<120> Verfahren zur Herstellung von L-Aminosäuren unter Verwendung von
   Stämmen der Familie Enterobacteriaceae
<130> 040163 BT
<160> 8
<170> PatentIn version 3.2
<210> 1
   <211> 27
   <212> DNA
   <213> künstliche Sequenz
<220>
   <221> Primer
   <222> (1)..(27)
   <223> P_lam1neu
<220>
   <221> Restriktionsschnittstelle
   <222> (1)..(6)
   <223> XbaI
<400> 1
   tctagagcct gtcacaggtg atgtgaa 27
<210> 2
   <211> 27
   <212> DNA
   <213> künstliche Sequenz
<220>
   <221> Primer
   <222> (1)..(27)
   <223> P_lam2neu
<220>
   <221> Restriktionsschnittstelle
   <222> (1)..(6)
   <223> HindIII
<400> 2
   aagcttacag ccgttgtagg cctgata 27
<210> 3
   <211> 1498
   <212> DNA
   <213> Escherichia coli
<220>
   <221> PCR-Produkt
   <222> (1)..(1498)
<223> lamB PCR-Produkt
<220>
   <221> primer_bind
   <222> (1)..(27)
   <223> Primer P_lam1neu
<220>
   <221> CDS
   <222> (57)..(1397)
   <223> lamB Kodierbereich
<220>
   <221> primer_bind
   <222> (1472)..(1498)
   <223> Primer P_lam2neu
<400> 3
   tctagagcct gtcacaggtg atgtgaaaaa agaaaagcaa tgactcagga gataga atg      59
   atg att act ctg cgc aaa ctt cct ctg gcg gtt gcc gtc gca gcg ggc       107
   gta atg tct gct cag gca atg gct gtt gat ttc cac ggc tat gca cgt       155
   tcc ggt att ggt tgg aca ggt agc ggc ggt gaa caa cag tgt ttc cag       203
   act acc ggt gct caa agt aaa tac cgt ctt ggc aac gaa tgt gaa act       251
   tat gct gaa tta aaa ttg ggt cag gaa gtg tgg aaa gag ggc gat aag       299
   agc ttc tat ttc gac act aac gtg gcc tat tcc gtc gca caa cag aat       347
   gac tgg gaa gct acc gat ccg gcc ttc cgt gaa gca aac gtg cag ggt       395
   aaa aac ctg atc gaa tgg ctg cca ggc tcc acc atc tgg gca ggt aag       443
   cgc ttc tac caa cgt cat gac gtt cat atg atc gac ttc tac tac tgg       491
   gat att tct ggt cct ggt gcc ggt ctg gaa aac atc gat gtt ggc ttc       539
   ggt aaa ctc tct ctg gca gca acc cgc tcc tct gaa gct ggt ggt tct       587
   tcc tct ttc gcc agc aac aat att tat gac tat acc aac gaa acc gcg      635
   aac gac gtt ttc gat gtg cgt tta gcg cag atg gaa atc aac ccg ggc      683
   ggc aca tta gaa ctg ggt gtc gac tac ggt cgt gcc aac ttg cgt gat      731
   aac tat cgt ctg gtt gat ggc gca tcg aaa gac ggc tgg tta ttc act      779
   gct gaa cat act cag agt gtc ctg aag ggc ttt aac aag ttt gtt gtt      827
   cag tac gct act gac tcg atg acc tcg cag ggt aaa ggg ctg tcg cag      875
   ggt tct ggc gtt gca ttt gat aac gaa aaa ttt gcc tac aat atc aac      923
   aac aac ggt cac atg ctg cgt atc ctc gac cac ggt gcg atc tcc atg      971
   ggc gac aac tgg gac atg atg tac gtg ggt atg tac cag gat atc aac     1019
   tgg gat aac gac aac ggc acc aag tgg tgg acc gtc ggt att cgc ccg     1067
   atg tac aag tgg acg cca atc atg agc acc gtg atg gaa atc ggc tac     1115
   gac aac gtc gaa tcc cag cgc acc ggc gac aag aac aat cag tac aaa     1163
   att acc ctc gca caa caa tgg cag gct ggc gac agc atc tgg tca cgc     1211
   ccg gct att cgt gtc ttc gca acc tac gcc aag tgg gat gag aaa tgg     1259
   ggt tac gac tac acc ggt aac gct gat aac aac gcg aac ttc ggc aaa     1307
   gcc gtt cct gct gat ttc aac ggc ggc agc ttc ggt cgt ggc gac agc     1355
   gac gag tgg acc ttc ggt gcc cag atg gaa atc tgg tgg taa                1397
   tagcaaaacc tgggccggat aaggcgttta cgccgcattc ggcaaccaac gcctgatgcg   1457
   acgcttgcgc gtcttatcag gcctacaacg gctgtaagct t                          1498
<210> 4
   <211> 446
   <212> PRT
   <213> Escherichia coli
<400> 4
<210> 5
   <211> 1341
   <212> DNA
   <213> Shigella flexneri
<220>
   <221> CDS
   <222> (1) .. (1341)
   lamB Kodierbereich
<400> 5
   atg atg att act ctg cgc aaa ctt cct ctg gcg gtt gcc gtc gca gcg       48
   Met Met Ile Thr Leu Arg Lys Leu Pro Leu Ala Val Ala Val Ala Ala
   1               5                   10                  15
   ggc gta atg tct gct cag gca atg gct gtt gat ttc cac ggc tat gca       96
   Gly Val Met Ser Ala Gln Ala Met Ala Val Asp Phe His Gly Tyr Ala
               20                  25                  30
   cgt tcc ggt att ggc tgg aca ggt agc ggc ggt gaa caa cag tgt ttc      144
   Arg Ser Gly Ile Gly Trp Thr Gly Ser Gly Gly Glu Gln Gln Cys Phe
           35                  40                  45
   cag act acc ggt gct caa agt aaa tac cgt ctt ggc aac gaa tgt gaa      192
   Gln Thr Thr Gly Ala Gln Ser Lys Tyr Arg Leu Gly Asn Glu Cys Glu
       50                  55                  60
   act tat gct gaa tta aaa ttg ggt cag gaa gtg tgg aaa gag ggc gat      240
   Thr Tyr Ala Glu Leu Lys Leu Gly Gln Glu Val Trp Lys Glu Gly Asp
   65                  70                  75                  80
   aag agc ttc tat ttc gac act aac gtg gcc tat tcc gtc gcg caa cag      288
   Lys Ser Phe Tyr Phe Asp Thr Asn Val Ala Tyr Ser Val Ala Gln Gln
                   85                  90                  95
   aat gac tgg gaa gct acc gat ccg gcc ttc cgt gaa gca aac gtg cag      336
   Asn Asp Trp Glu Ala Thr Asp Pro Ala Phe Arg Glu Ala Asn Val Gln
               100                 105                 110
   gga aaa aac ctg atc gaa tgg ctg cca ggt tcc acc atc tgg gca ggt      384
   Gly Lys Asn Leu Ile Glu Trp Leu Pro Gly Ser Thr Ile Trp Ala Gly
           115                 120                 125
   aag cgc ttc tac caa cgt cat gac gtt cat atg atc gac ttc tac tac      432
   Lys Arg Phe Tyr Gln Arg His Asp Val His Met Ile Asp Phe Tyr Tyr
       130                 135                 140
   tgg gat att tct ggt cct ggt gcc ggt ctg gaa aac atc gat gtt ggc      480
   Trp Asp Ile Ser Gly Pro Gly Ala Gly Leu Glu Asn Ile Asp Val Gly
   145                 150                 155                 160
   ttc ggt aaa ctc tct ctg gca gca acc cgc tcc tct gaa gct ggt ggt      528
   Phe Gly Lys Leu Ser Leu Ala Ala Thr Arg Ser Ser Glu Ala Gly Gly
                   165                 170                 175
   tct tcc tct ttt gcc agc aac aat att tat gac tat acc aac gaa acc      576
   Ser Ser Ser Phe Ala Ser Asn Asn Ile Tyr Asp Tyr Thr Asn Glu Thr
               180                 185                 190
   gcg aac gac gtt ttc gat gtg cgt tta gcg cag atg gaa atc aac ccg      624
   Ala Asn Asp Val Phe Asp Val Arg Leu Ala Gln Met Glu Ile Asn Pro
           195                 200                 205
   ggc ggc aca tta gaa ctg ggt gtc gac tac ggt cgt gcc aac ctg cgt      672
   Gly Gly Thr Leu Glu Leu Gly Val Asp Tyr Gly Arg Ala Asn Leu Arg
       210                 215                 220
   gat aac tat cgt ctg gtt gat ggc gca tcg aaa gac ggc tgg tta ttc      720
   Asp Asn Tyr Arg Leu Val Asp Gly Ala Ser Lys Asp Gly Trp Leu Phe
   225                 230                 235                 240
   act gct gaa cat act cag agt gtc ctg aag ggc ttt aac aag ttt gtt      768
   Thr Ala Glu His Thr Gln Ser Val Leu Lys Gly Phe Asn Lys Phe Val
                   245                 250                 255
   gtt cag tac gct act gac tcg atg acc tcg cag ggt aaa ggt ctg tcg      816
   Val Gln Tyr Ala Thr Asp Ser Met Thr Ser Gln Gly Lys Gly Leu Ser
               260                 265                 270
   cag ggt tct ggc gtc gcg ttt gat aac gaa aaa ttt gcc tac aat atc      864
   Gln Gly Ser Gly Val Ala Phe Asp Asn Glu Lys Phe Ala Tyr Asn Ile
           275                 280                 285
   aac aac aac ggt cac atg ctg cgt atc ctc gac cac ggt gcg atc tcc      912
   Asn Asn Asn Gly His Met Leu Arg Ile Leu Asp His Gly Ala Ile Ser
       290                 295                 300
   atg ggc gac aac tgg gac atg atg tac gtg ggt atg tac cag gat atc      960
   Met Gly Asp Asn Trp Asp Met Met Tyr Val Gly Met Tyr Gln Asp Ile
   305                 310                 315                 320
   aac tgg gat aac gac aac ggc acc aag tgg tgg acc gtt ggt att cgc     1008
   Asn Trp Asp Asn Asp Asn Gly Thr Lys Trp Trp Thr Val Gly Ile Arg
                   325                 330                 335
   ccg atg tac aag tgg acg cca atc atg agc acc gtg atg gaa atc ggc     1056
   Pro Met Tyr Lys Trp Thr Pro Ile Met Ser Thr Val Met Glu Ile Gly
               340                 345                 350
   tac gac aac gtc gaa tcc cag cgc acc ggc gac aag aac aat cag tac     1104
   Tyr Asp Asn Val Glu Ser Gln Arg Thr Gly Asp Lys Asn Asn Gln Tyr
           355                 360                 365
   aaa att acc ctc gca caa caa tgg cag gct ggc gac agc atc tgg tca     1152
   Lys Ile Thr Leu Ala Gln Gln Trp Gln Ala Gly Asp Ser Ile Trp Ser
       370                 375                 380
   cgc ccg gct att cgt gtc ttc gca acc tac gcc aag tgg gat gag aaa     1200
   Arg Pro Ala Ile Arg Val Phe Ala Thr Tyr Ala Lys Trp Asp Glu Lys
   385                 390                 395                 400
   tgg ggt tac gac tac aac ggc gat agc aag gtt aac ccg aac tac ggc     1248
   Trp Gly Tyr Asp Tyr Asn Gly Asp Ser Lys Val Asn Pro Asn Tyr Gly
                   405                 410                 415
   aaa gcc gtt cct gct gat ttc aac ggc ggc agc ttc ggt cgt ggc gac     1296
   Lys Ala Val Pro Ala Asp Phe Asn Gly Gly Ser Phe Gly Arg Gly Asp
               420                 425                 430
   agc gac gag tgg acc ttc ggt gcc cag atg gaa atc tgg tgg taa         1341
   Ser Asp Glu Trp Thr Phe Gly Ala Gln Met Glu Ile Trp Trp
           435                 440                 445

<210> 6
   <211> 446
   <212> PRT
   <213> Shigella flexneri
<400> 6
   Met Met Ile Thr Leu Arg Lys Leu Pro Leu Ala Val Ala Val Ala Ala
   1               5                   10                  15
   Gly Val Met Ser Ala Gln Ala Met Ala Val Asp Phe His Gly Tyr Ala
               20                  25                  30
   Arg Ser Gly Ile Gly Trp Thr Gly Ser Gly Gly Glu Gln Gln Cys Phe
           35                  40                  45
   Gln Thr Thr Gly Ala Gln Ser Lys Tyr Arg Leu Gly Asn Glu Cys Glu
       50                  55                  60
   Thr Tyr Ala Glu Leu Lys Leu Gly Gln Glu Val Trp Lys Glu Gly Asp
   65                  70                  75                  80
   Lys Ser Phe Tyr Phe Asp Thr Asn Val Ala Tyr Ser Val Ala Gln Gln
                   85                  90                  95
   Asn Asp Trp Glu Ala Thr Asp Pro Ala Phe Arg Glu Ala Asn Val Gln
               100                 105                 110
   Gly Lys Asn Leu Ile Glu Trp Leu Pro Gly Ser Thr Ile Trp Ala Gly
           115                 120                 125
   Lys Arg Phe Tyr Gln Arg His Asp Val His Met Ile Asp Phe Tyr Tyr
       130                 135                 140
   Trp Asp Ile Ser Gly Pro Gly Ala Gly Leu Glu Asn Ile Asp Val Gly
   145                 150                 155                 160
   Phe Gly Lys Leu Ser Leu Ala Ala Thr Arg Ser Ser Glu Ala Gly Gly
                   165                 170                 175
   Ser Ser Ser Phe Ala Ser Asn Asn Ile Tyr Asp Tyr Thr Asn Glu Thr
               180                 185                 190
   Ala Asn Asp Val Phe Asp Val Arg Leu Ala Gln Met Glu Ile Asn Pro
           195                 200                 205
   Gly Gly Thr Leu Glu Leu Gly Val Asp Tyr Gly Arg Ala Asn Leu Arg
       210                 215                 220
   Asp Asn Tyr Arg Leu Val Asp Gly Ala Ser Lys Asp Gly Trp Leu Phe
   225                 230                 235                 240
   Thr Ala Glu His Thr Gln Ser Val Leu Lys Gly Phe Asn Lys Phe Val
                   245                 250                 255
   Val Gln Tyr Ala Thr Asp Ser Met Thr Ser Gln Gly Lys Gly Leu Ser
               260                 265                 270
   Gln Gly Ser Gly Val Ala Phe Asp Asn Glu Lys Phe Ala Tyr Asn Ile
           275                 280                 285
   Asn Asn Asn Gly His Met Leu Arg Ile Leu Asp His Gly Ala Ile Ser
       290                 295                 300
   Met Gly Asp Asn Trp Asp Met Met Tyr Val Gly Met Tyr Gln Asp Ile
   305                 310                 315                 320
   Asn Trp Asp Asn Asp Asn Gly Thr Lys Trp Trp Thr Val Gly Ile Arg
                   325                 330                 335
   Pro Met Tyr Lys Trp Thr Pro Ile Met Ser Thr Val Met Glu Ile Gly
               340                 345                 350
   Tyr Asp Asn Val Glu Ser Gln Arg Thr Gly Asp Lys Asn Asn Gln Tyr
           355                 360                 365
   Lys Ile Thr Leu Ala Gln Gln Trp Gln Ala Gly Asp Ser Ile Trp Ser
       370                 375                 380
   Arg Pro Ala Ile Arg Val Phe Ala Thr Tyr Ala Lys Trp Asp Glu Lys
   385                 390                 395                 400
   Trp Gly Tyr Asp Tyr Asn Gly Asp Ser Lys Val Asn Pro Asn Tyr Gly
                   405                 410                 415
   Lys Ala Val Pro Ala Asp Phe Asn Gly Gly Ser Phe Gly Arg Gly Asp
               420                 425                 430
   Ser Asp Glu Trp Thr Phe Gly Ala Gln Met Glu Ile Trp Trp
           435                 440                 445
<210> 7
   <211> 1359
   <212> DNA
   <213> Salmonella typhimurium
<220>
   <221> CDS
<222> (1)..(1359)
   <223> lamB Kodierbereich
<400> 7
   atg atg att act ctg cgc aaa ctc cca ctg gcg gtt gct gtc gca gcg      48
   Met Met Ile Thr Leu Arg Lys Leu Pro Leu Ala Val Ala Val Ala Ala
   1               5                   10                  15
   ggc gta atg tcc gct cag gca atg gct gtc gat ttc cac ggt tac gcc      96
   Gly Val Met Ser Ala Gln Ala Met Ala Val Asp Phe His Gly Tyr Ala
               20                  25                  30
   cgt tcc ggt att ggc tgg acg gga agc ggc ggc gaa caa cag tgt ttc     144
   Arg Ser Gly Ile Gly Trp Thr Gly Ser Gly Gly Glu Gln Gln Cys Phe
           35                  40                  45
   cag gca acg ggt gcc caa agt aaa tac cgt ctc ggt aac gaa tgt gaa     192
   Gln Ala Thr Gly Ala Gln Ser Lys Tyr Arg Leu Gly Asn Glu Cys Glu
       50                  55                  60
   acc tat gcg gaa ctg aaa ctg ggc cag gaa gtg tgg aaa gag ggc gat     240
   Thr Tyr Ala Glu Leu Lys Leu Gly Gln Glu Val Trp Lys Glu Gly Asp
   65                  70                  75                  80
   aag agc ttc tat ttc gac acc aac gtc gcc tat tcg gtt aac cag cag     288
   Lys Ser Phe Tyr Phe Asp Thr Asn Val Ala Tyr Ser Val Asn Gln Gln
                   85                  90                  95
   aac gac tgg gaa tcg acc gat ccc gcc ttc cgc gaa gcg aac gtg cag     336
   Asn Asp Trp Glu Ser Thr Asp Pro Ala Phe Arg Glu Ala Asn Val Gln
               100                 105                 110
   ggt aaa aac ctg att gaa tgg ctg ccg ggc tct acc atc tgg gcc ggt     384
   Gly Lys Asn Leu Ile Glu Trp Leu Pro Gly Ser Thr Ile Trp Ala Gly
           115                 120                 125
   aag cgc ttc tat cag cgt cat gac gta cac atg atc gac ttc tac tac     432
   Lys Arg Phe Tyr Gln Arg His Asp Val His Met Ile Asp Phe Tyr Tyr
       130                 135                 140
   tgg gat att tca ggt cct ggc gca ggt atc gaa aat atc gat ctg ggc     480
   Trp Asp Ile Ser Gly Pro Gly Ala Gly Ile Glu Asn Ile Asp Leu Gly
   145                 150                 155                 160
   ttc ggt aag ctt tca ctg gcg gcg acc cgg tct act gaa gcg ggc ggc     528
   Phe Gly Lys Leu Ser Leu Ala Ala Thr Arg Ser Thr Glu Ala Gly Gly
                   165                 170                 175
   tct tac acc ttc agc agc cag aat att tat gat gaa gtg aaa gat acc     576
   Ser Tyr Thr Phe Ser Ser Gln Asn Ile Tyr Asp Glu Val Lys Asp Thr
               180                 185                 190
   gct aac gac gtc ttt gac gta cgt ctg gct ggt ctg caa acg aac ccg     624
   Ala Asn Asp Val Phe Asp Val Arg Leu Ala Gly Leu Gln Thr Asn Pro
           195                 200                 205
   gac ggc gta ctg gag cta ggc gtt gat tac ggt cgc gcc aat acg acc     672
   Asp Gly Val Leu Glu Leu Gly Val Asp Tyr Gly Arg Ala Asn Thr Thr
       210                 215                 220
   gat ggt tat aag ctg gct gat ggg gca tcg aaa gac ggc tgg atg ttc     720
   Asp Gly Tyr Lys Leu Ala Asp Gly Ala Ser Lys Asp Gly Trp Met Phe
   225                 230                 235                 240
   acc gcc gaa cac acg caa agc atg ttg aaa ggc tat aac aag ttt gtc      768
   Thr Ala Glu His Thr Gln Ser Met Leu Lys Gly Tyr Asn Lys Phe Val
                   245                 250                 255
   gtg caa tat gcc acc gat gcc atg acc acg cag ggt aaa ggc cag gcg      816
   Val Gln Tyr Ala Thr Asp Ala Met Thr Thr Gln Gly Lys Gly Gln Ala
               260                 265                 270
   cgc ggt tcc gac ggt tct tca tct ttc act gaa gaa ttg tct gat gga      864
   Arg Gly Ser Asp Gly Ser Ser Ser Phe Thr Glu Glu Leu Ser Asp Gly
           275                 280                 285
   acc aaa att aat tac gcc aat aag gtc atc aat aat aat ggc aat atg      912
   Thr Lys Ile Asn Tyr Ala Asn Lys Val Ile Asn Asn Asn Gly Asn Met
       290                 295                 300
   tgg cgt att ttg gat cat ggc gcc atc tcg ctt ggt gat aaa tgg gat      960
   Trp Arg Ile Leu Asp His Gly Ala Ile Ser Leu Gly Asp Lys Trp Asp
   305                 310                 315                 320
   ttg atg tac gtc ggt atg tac cag aat atc gat tgg gat aat aac ctg     1008
   Leu Met Tyr Val Gly Met Tyr Gln Asn Ile Asp Trp Asp Asn Asn Leu
                   325                 330                 335
   ggt act gag tgg tgg acc gtg ggt gta cgt cca atg tac aag tgg acg     1056
   Gly Thr Glu Trp Trp Thr Val Gly Val Arg Pro Met Tyr Lys Trp Thr
               340                 345                 350
   cca atc atg agc acc ctg ctg gaa gtc ggc tac gac aac gtg aaa tct     1104
   Pro Ile Met Ser Thr Leu Leu Glu Val Gly Tyr Asp Asn Val Lys Ser
           355                 360                 365
   cag cag acc ggc gat cgt aac aat caa tat aaa atc acc ctg gcg caa     1152
   Gln Gln Thr Gly Asp Arg Asn Asn Gln Tyr Lys Ile Thr Leu Ala Gln
       370                 375                 380
   cag tgg cag gcg ggc gac agc atc tgg tcg cgt ccg gct atc cgt att     1200
   Gln Trp Gln Ala Gly Asp Ser Ile Trp Ser Arg Pro Ala Ile Arg Ile
   385                 390                 395                 400
   ttc gcc acc tac gcg aaa tgg gat gag aaa tgg ggc tat atc aaa gac     1248
   Phe Ala Thr Tyr Ala Lys Trp Asp Glu Lys Trp Gly Tyr Ile Lys Asp
                   405                 410                 415
   ggc gat aac att tcc cgt tat gcc gca gcg act aac tcc ggc att tcc     1296
   Gly Asp Asn Ile Ser Arg Tyr Ala Ala Ala Thr Asn Ser Gly Ile Ser
               420                 425                 430
   acc aac agc cgt ggc gat agc gat gag tgg acc ttc ggc gcc cag atg     1344
   Thr Asn Ser Arg Gly Asp Ser Asp Glu Trp Thr Phe Gly Ala Gln Met
           435                 440                 445
   gaa atc tgg tgg taa                                                 1359
   Glu Ile Trp Trp
       450
<210> 8
   <211> 452
   <212> PRT
<213> Salmonella typhimurium
<400> 8
   Met Met Ile Thr Leu Arg Lys Leu Pro Leu Ala Val Ala Val Ala Ala
   1 5 10 15
   Gly Val Met Ser Ala Gln Ala Met Ala Val Asp Phe His Gly Tyr Ala
   20 25 30
   Arg Ser Gly Ile Gly Trp Thr Gly Ser Gly Gly Glu Gln Gln Cys Phe
   35 40 45
   Gln Ala Thr Gly Ala Gln Ser Lys Tyr Arg Leu Gly Asn Glu Cys Glu
   50 55 60
   Thr Tyr Ala Glu Leu Lys Leu Gly Gln Glu Val Trp Lys Glu Gly Asp
   65 70 75 80
   Lys Ser Phe Tyr Phe Asp Thr Asn Val Ala Tyr Ser Val Asn Gln Gln
   85 90 95
   Asn Asp Trp Glu Ser Thr Asp Pro Ala Phe Arg Glu Ala Asn Val Gln
   100 105 110
   Gly Lys Asn Leu Ile Glu Trp Leu Pro Gly Ser Thr Ile Trp Ala Gly
   115 120 125
   Lys Arg Phe Tyr Gln Arg His Asp Val His Met Ile Asp Phe Tyr Tyr
   130 135 140
   Trp Asp Ile Ser Gly Pro Gly Ala Gly Ile Glu Asn Ile Asp Leu Gly
   145 150 155 160
   Phe Gly Lys Leu Ser Leu Ala Ala Thr Arg Ser Thr Glu Ala Gly Gly
   165 170 175
   Ser Tyr Thr Phe Ser Ser Gln Asn Ile Tyr Asp Glu Val Lys Asp Thr
   180 185 190
   Ala Asn Asp Val Phe Asp Val Arg Leu Ala Gly Leu Gln Thr Asn Pro
   195 200 205
   Asp Gly Val Leu Glu Leu Gly Val Asp Tyr Gly Arg Ala Asn Thr Thr
   210 215 220
   Asp Gly Tyr Lys Leu Ala Asp Gly Ala Ser Lys Asp Gly Trp Met Phe
   225 230 235 240
   Thr Ala Glu His Thr Gln Ser Met Leu Lys Gly Tyr Asn Lys Phe Val
   245 250 255
   Val Gln Tyr Ala Thr Asp Ala Met Thr Thr Gln Gly Lys Gly Gln Ala
               260                 265                 270
   Arg Gly Ser Asp Gly Ser Ser Ser Phe Thr Glu Glu Leu Ser Asp Gly
           275                 280                 285
   Thr Lys Ile Asn Tyr Ala Asn Lys Val Ile Asn Asn Asn Gly Asn Met
       290                 295                 300
   Trp Arg Ile Leu Asp His Gly Ala Ile Ser Leu Gly Asp Lys Trp Asp
   305                 310                 315                 320
   Leu Met Tyr Val Gly Met Tyr Gln Asn Ile Asp Trp Asp Asn Asn Leu
                   325                 330                 335
   Gly Thr Glu Trp Trp Thr Val Gly Val Arg Pro Met Tyr Lys Trp Thr
               340                 345                 350
   Pro Ile Met Ser Thr Leu Leu Glu Val Gly Tyr Asp Asn Val Lys Ser
           355                 360                 365
   Gln Gln Thr Gly Asp Arg Asn Asn Gln Tyr Lys Ile Thr Leu Ala Gln
       370                 375                 380
   Gln Trp Gln Ala Gly Asp Ser Ile Trp Ser Arg Pro Ala Ile Arg Ile
   385                 390                 395                 400
   Phe Ala Thr Tyr Ala Lys Trp Asp Glu Lys Trp Gly Tyr Ile Lys Asp
                   405                 410                 415
   Gly Asp Asn Ile Ser Arg Tyr Ala Ala Ala Thr Asn Ser Gly Ile Ser
               420                 425                 430
   Thr Asn Ser Arg Gly Asp Ser Asp Glu Trp Thr Phe Gly Ala Gln Met
           435                 440                 445
   Glu Ile Trp Trp
       450

## Beschreibung

Diese Erfindung betrifft ein Verfahren zur Herstellung von L-Aminosäuren, insbesondere L-Threonin, unter Verwendung von rekombinanten Mikroorganismen der Familie Enterobacteriaceae, in denen das lamB-Gen oder für dessen Genprodukt Maltoporin kodierende Nukleotidsequenzen verstärkt, insbesondere überexprimiert wird bzw. werden, und diese Mikroorganismen.

### Stand der Technik

L-Aminosäuren, insbesondere L-Threonin, finden in der Humanmedizin und in der pharmazeutischen Industrie, in der Lebensmittelindustrie und ganz besonders in der Tierernährung Anwendung.

Es ist bekannt, dass L-Aminosäuren durch Fermentation von Stämmen der Enterobacteriaceae, insbesondere Escherichia coli (E. coli) und Serratia marcescens, hergestellt werden. Wegen der großen Bedeutung wird ständig an der Verbesserung der Herstellverfahren gearbeitet. Verfahrensverbesserungen können fermentationstechnische Maßnahmen, wie z.B. Rührung und Versorgung mit Sauerstoff, oder die Zusammensetzung der Nährmedien wie z.B. die Zuckerkonzentration während der Fermentation, oder die Aufarbeitung zur Produktform, durch z.B. Ionenaustauschchromatographie, oder die intrinsischen Leistungseigenschaften des Mikroorganismus selbst betreffen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite wie z.B. das Threonin-Analogon α-Amino-β-Hydroxyvaleriansäure (AHV) oder auxotroph für regulatorisch bedeutsame Metabolite sind und L-Aminosäuren wie z.B. L-Threonin produzieren.

Seit einigen Jahren werden ebenfalls Methoden der rekombinanten DNA-Technik zur Stammverbesserung L-Aminosäuren produzierender Stämme der Familie Enterobacteriaceae eingesetzt, indem man einzelne Aminosäure-Biosynthesegene amplifiziert und die Auswirkung auf die Produktion untersucht. Zusammenfassende Informationen zur Zell- und Molekularbiologie von Escherichia coli und Salmonella sind in Neidhardt (ed): Escherichia coli and Salmonella, Cellular and Molecular Biology, 2nd edition, ASM Press, Washington, D.C., USA, (1996) zu finden.

### Aufgabe der Erfindung

Die Erfinder haben sich die Aufgabe gestellt, neue Maßnahmen zur verbesserten fermentativen Herstellung von L-Aminosäuren, insbesondere L-Threonin, bereitzustellen.

### Beschreibung der Erfindung

Gegenstand der Erfindung sind rekombinante Mikroorganismen der Familie Enterobacteriaceae, die ein verstärktes oder überexprimiertes lamB-Gen enthalten, das für ein Polypeptid mit der Aktivität des Maltoporin LamB kodiert, und die in verbesserter Weise L-Aminosäuren, insbesondere L-Threonin, produzieren.

Als Ausgangspunkt für den Vergleich dienen jeweils die nicht rekombinanten Mikroorganismen, die kein verstärktes lamB-Gen enthalten.

Zu diesen Mikroorganismen gehören insbesondere Mikroorganismen der Familie Enterobacteriaceae, in denen ein Polynukleotid verstärkt wird, das für ein Polypeptid kodiert, das zu mindestens 90 %, insbesondere zu mindestens 95 %, bevorzugt 99 % identisch ist mit einer Aminosäuresequenz, ausgewählt aus der Gruppe SEQ ID No. 4, SEQ ID No. 6, und SEQ ID No. 8, wobei das Polypeptid die Aktivität des Maltoporin LamB besitzt.

Bevorzugt sind Aminosäuresequenzen, die identisch sind mit denen aus SEQ ID No. 4, SEQ ID No. 6 oder SEQ ID No. 8.

Die genannten Mikroorganismen enthalten verstärkte oder überexprimierte Polynukleotide, ausgewählt aus der Gruppe:
- a): Polynukleotid mit der Nukleotidsequenz SEQ ID No. 3, SEQ ID No. 5 oder SEQ ID No. 7;
- b): Polynukleotid mit einer Nukleotidsequenz, die der SEQ ID No. 3, SEQ ID No. 5 oder SEQ ID No. 7 im Rahmen der Degeneration des genetischen Codes entspricht;
- c): Polynukleotidsequenz mit einer Sequenz, die mit der zur Sequenz SEQ ID No. 3, SEQ ID No. 5 oder SEQ ID No. 7 komplementären Sequenz unter stringenten Bedingungen hybridisiert;
- d): Polynukleotid mit einer Sequenz SEQ ID No. 3, SEQ ID No. 5 oder SEQ ID No. 7, die funktionsneutrale Sinnmutanten enthält,
wobei die Polynukleotide für das Genprodukt Maltoporin kodieren (s. u.).

Gegenstand der Erfindung ist ebenso ein Verfahren zur fermentativen Herstellung von L-Aminosäuren, insbesondere L-Threonin, unter Verwendung von rekombinanten Mikroorganismen der Familie Enterobacteriaceae, die insbesondere bereits L-Aminosäuren produzieren, und in denen mindestens das lamB-Gen oder für dessen Genprodukt Maltoporin LamB kodierende Nukleotidsequenzen verstärkt wird bzw. werden.

Bevorzugt werden die beschriebenen Mikroorganismen eingesetzt.

werden im folgenden L-Aminosäuren oder Aminosäuren erwähnt, sind damit eine oder mehrere Aminosäuren einschließlich ihrer Salze, ausgewählt aus der Gruppe L-Asparagin, L-Threonin, L-Serin, L-Glutamat, L-Glycin, L-Alanin, L-Cystein, L-Valin, L-Methionin, L-Prolin, L-Isoleucin,. L-Leucin, L-Tyrosin, L-Phenylalanin, L-Histidin, L-Lysin, L-Tryptophan, L-Arginin und L-Homoserin gemeint. Besonders bevorzugt ist L-Threonin.

Der Begriff "Verstärkung" beschreibt in diesem Zusammenhang die Erhöhung der intrazellulären Aktivität oder Konzentration eines oder mehrerer Enzyme oder Proteine in einem Mikroorganismus, die durch die entsprechende DNA kodiert werden, indem man beispielsweise die Kopienzahl des Gens bzw. der Gene um mindestens eine (1) Kopie erhöht, einen starken Promotor funktionell mit dem Gen verknüpft oder ein Gen oder Allel verwendet, das für ein entsprechendes Enzym bzw. Protein mit einer hohen Aktivität kodiert und gegebenenfalls diese Maßnahmen kombiniert.

Durch die Maßnahmen der Verstärkung, insbesondere Überexpression, wird die Aktivität oder Konzentration des entsprechenden Proteins im allgemeinen um mindestens 10%, 25%, 50%, 75%, 100%, 150%, 200%, 300%, 400% oder 500%, maximal bis 1000% oder 2000% bezogen auf die des Wildtyp-Proteins beziehungsweise auf die Aktivität oder Konzentration des Proteins im nicht rekombinanten Mikroorganismus erhöht. Als nicht rekombinanter Mikroorganismus oder Elternstamm (parent strain) wird der Mikroorganismus verstanden, an dem die erfinderischen Maßnahmen durchgeführt werden.

Gegenstand der Erfindung ist ein Verfahren zur Herstellung von L-Aminosäuren durch Fermentation von rekombinanten Mikroorganismen der Familie Enterobacteriaceae, dadurch gekennzeichnet, dass man
- a): die die gewünschte L-Aminosäure produzierenden Mikroorganismen, in denen man das lamB-Gen oder für dessen Genprodukt kodierende Nukleotidsequenz verstärkt, insbesondere überexprimiert, in einem Medium kultiviert unter Bedingungen, bei denen die gewünschte L-Aminosäure im Medium oder in den Zellen angereichert wird und,
- b): die gewünschte L-Aminosäure isoliert, wobei gegebenenfalls Bestandteile der Fermentationsbrühe und/oder die Biomasse in ihrer Gesamtheit oder Anteilen (≥ 0 bis 100 %) im isolierten Produkt verbleiben oder vollständig entfernt werden.

Die insbesondere rekombinanten Mikroorganismen mit einem verstärkten oder überexprimierten lamB-Gen, die ebenfalls Gegenstand der vorliegenden Erfindung sind, können L-Aminosäuren aus Glucose, Saccharose, Lactose, Fructose, Maltose, Melasse, gegebenenfalls Stärke, gegebenenfalls Cellulose oder aus Glycerin und Ethanol herstellen. Es handelt sich um Vertreter der Familie Enterobacteriaceae, ausgewählt aus den Gattungen Escherichia, Erwinia, Providencia und Serratia. Die Gattungen Escherichia und Serratia werden bevorzugt. Bei der Gattung Escherichia ist insbesondere die Art Escherichia coli und bei der Gattung Serratia insbesondere die Art Serratia marcescens zu nennen.

Rekombinante Mikroorganismen werden im Allgemeinen durch Transformation, Transduktion, Konjugation, oder einer Kombination dieser Methoden, mit einem Vektor erzeugt, der das gewünschte Gen, ein Allel dieses Gens oder Teile davon oder einen die Exprimierung des Gens verstärkenden Promotor enthält.

Geeignete, insbesondere L-Threonin produzierende Stämme der Gattung Escherichia, insbesondere der Art Escherichia coli sind beispielsweise
- Escherichia coli H4581 (EP 0 301 572)
- Escherichia coli.KY10935 (Bioscience Biotechnology and Biochemistry 61(11):1877-1882 (1997)
- Escherichia coli VNIIgenetika MG442 (US-A-4278,765)
- Escherichia coli VNIIgenetika M1 (US-A-4,321,325)
- Escherichia coli VNIIgenetika 472T23 (US-A-5,631,157)
- Escherichia coli BKIIM B-3996 (US-A-5,175,107)
- Escherichia coli kat 13 (WO 98/04715)
- Escherichia coli KCCM-10132 (WO 00/09660) Geeignete L-Threonin produzierende Stämme der Gattung Serratia, insbesondere der Art Serratia marcescens sind beispielsweise
- Serratia marcescens HNr21 (Applied and Environmental Microbiology 38(6): 1045-1051 (1979))
- Serratia marcescens TLr156 (Gene 57(2-3): 151-158 (1987))
- Serratia marcescens T-2000 (Applied Biochemistry and Biotechnology 37(3): 255-265 (1992))

L-Threonin produzierende Stämme aus der Familie der Enterobacteriaceae besitzen bevorzugt, unter anderen, ein oder mehrere der genetischen bzw. phänotypischen Merkmale ausgewählt aus der Gruppe: Resistenz gegen α-Amino-β-Hydroxyvaleriansäure, Resistenz gegen Thialysin, Resistenz gegen Ethionin, Resistenz gegen α-Methylserin, Resistenz gegen Diaminobernsteinsäure, Resistenz gegen α-Aminobuttersäure, Resistenz gegen Borrelidin, Resistenz gegen Cyclopentan-Carboxylsäure, Resistenz gegen Rifampicin, Resistenz gegen Valin-Analoga wie beispielsweise Valinhydroxamat, Resistenz gegen Purinanaloga wie beispielsweise 6-Dimethylaminopurin, Bedürftigkeit für L-Methionin, gegebenenfalls partielle und kompensierbare Bedürftigkeit für L-Isoleucin, Bedürftigkeit für meso-Diaminopimelinsäure, Auxotrophie bezüglich

Threonin-haltiger Dipeptide, Resistenz gegen L-Threonin, Resistenz gegen Threonin-Raffinat, Resistenz gegen L-Homoserin, Resistenz gegen L-Lysin, Resistenz gegen L-Methionin, Resistenz gegen L-Glutaminsäure, Resistenz gegen L-Aspartat, Resistenz gegen L-Leucin, Resistenz gegen L-Phenylalanin, Resistenz gegen L-Serin, Resistenz gegen L-Cystein, Resistenz gegen L-Valin, Empfindlichkeit gegenüber Fluoropyruvat, defekte Threonin-Dehydrogenase, gegebenenfalls Fähigkeit zur Saccharose-Verwertung, Verstärkung des Threonin-Operons, Verstärkung der Homoserin-Dehydrogenase I-Aspartatkinase I bevorzugt der feed back resistenten Form, Verstärkung der Homoserinkinase, Verstärkung der Threoninsynthase, Verstärkung der Aspartatkinase, gegebenenfalls der feed back resistenten Form, Verstärkung der Aspartatsemialdehyd-Dehydrogenase, Verstärkung der Phosphoenolpyruvat-Carboxylase, gegebenenfalls der feed back resistenten Form, Verstärkung der Phosphoenolpyruvat-Synthase, Verstärkung der Transhydrogenase, Verstärkung des RhtB-Genproduktes, Verstärkung des RhtC-Genproduktes, Verstärkung des YfiK-Genproduktes, Verstärkung einer Pyruvat-Carboxylase, und Abschwächung der Essigsäurebildung.

Es wurde gefunden, dass Mikroorganismen der Familie Enterobacteriaceae nach Verstärkung, insbesondere Überexpression des Gens lamB, in verbesserter Weise L-Aminosäuren, insbesondere L-Threonin produzieren.

Die Nukleotidsequenzen der Gene oder offenen Leserahmen (ORF) von Escherichia coli gehören zum Stand der Technik und können der von Blattner et al. (Science 277: 1453-1462 (1997)) publizierten Genomsequenz von Escherichia coli entnommen werden. Es ist bekannt, dass durch wirtseigene Enzyme (Methionin-Aminipeptidase) die N-terminale Aminosäure Methionin abgespalten werden kann.

Aus den ebenfalls zur Familie Enterobacteriaceae gehörenden Shigella flexneri und Salmonella typhimirium sind die Nukleotidsequenzen für das lamB-Gen ebenso bekannt.

Das lamB-Gen von Escherichia coli wird unter anderem durch folgende Angaben beschrieben:
- Bezeichnung:: Das lamB-Gen aus Escherichia coli kodiert für das Maltoporin LamB, welches auch als Maltose/Maltodextrin- spezifisches porenbildendes Membranprotein (Porin) bezeichnet wird.
- Funktion:: Das Maltoporin LamB aus Escherichia coli ist ein trimerisches Protein, welches die Diffusion von Maltose und Maltodextrinen durch die äußere Membran enterischer Bakterien erleichtert bzw. ermöglicht und außerdem als ein unspezifisches Porin für kleine hydrophile Moleküle wie z.B. Glukose oder Trehalose wie auch als Zell- Oberflächen-Rezeptor (cell-surface receptor) für Phagen fungiert.
- Referenz:: Blattner et al.; Science 277(5331): 1453-1474 (1997),

Clement et al.; Cell 27: 507-514 (1981),
Schwartz; Methods of Enzymology 97: 100-112 (1983), Death et al.; Journal of Bacteriology 175: 1475-1483 (1993),
Klein et al.; Journal of Microbiology 175: 1682-1686 (1993),
Szmelcman et al.; Journal of Bacteriology 124: 112-118 (1975),
Benz et al.; Biochem. Biophys. Acta 1104: 299-307 (1992) Accession No.: AE000477
Alternativer Genname: b4036

Die Nukleinsäuresequenzen können den Datenbanken des National Center for Biotechnology Information (NCBI) der National Library of Medicine (Bethesda, MD, USA), der Nukleotidsequenz-Datenbank der European Molecular Biologies Laboratories (EMBL, Heidelberg, Deutschland bzw. Cambridge, UK) oder der DNA Datenbank von Japan (DDBJ, Mishima, Japan) entnommen werden.

Die Nukleotidsequenz des lamB-Gens von Escherichia coli findet sich in der SEQ ID No. 3 und die bekannten Sequenzen zum lamB-Gen von Shigella flexneri (AE015426) und Salmonella typhimurium (AE008897) unter den SEQ ID No. 5 bzw. SEQ ID No. 7. Die Aminosäuresequenzen der von diesen Leserahmen kodierten Proteine sind als SEQ ID No. 4, SEQ ID No. 6 bzw. SEQ ID No. 8 dargestellt.

Die in den angegebenen Textstellen beschriebenen Gene können erfindungsgemäß verwendet werden. Weiterhin können Allele der Gene verwendet werden, die sich aus der Degeneriertheit des genetischen Codes oder durch funktionsneutrale Sinnmutationen ("sense mutations") ergeben. Die Verwendung endogener Gene wird bevorzugt.

Unter "endogenen Genen" oder "endogenen Nukleotidsequenzen"-versteht man die in der Population einer Art vorhandenen Gene oder Allele beziehungsweise Nukleotidsequenzen.

Zu den geeigneten Allelen des lamB-Gens, welche funktionsneutrale Sinnmutationen enthalten, zählen unter anderem solche, die zu mindestens einem (1) konservativen Aminosäureaustausch in dem von ihnen kodierten Protein führen.

Bei den aromatischen Aminosäuren spricht man von konservativen Austauschen wenn Phenylalanin, Tryptophan und Tyrosin gegeneinander ausgetauscht werden. Bei den hydrophoben Aminosäuren spricht man von konservativen Austauschen wenn Leucin, Isoleucin und Valin gegeneinander ausgetauscht werden. Bei den polaren Aminosäuren spricht man von konservativen Austauschen wenn Glutamin und Asparagin gegeneinander ausgetauscht werden. Bei den basischen Aminosäuren spricht man von konservativen Austauschen wenn Arginin, Lysin und Histidin gegeneinander ausgetauscht werden. Bei den sauren Aminosäuren spricht man von konservativen Austauschen wenn Asparaginsäure und Glutaminsäure gegeneinander ausgetauscht werden. Bei den Hydroxyl-Gruppen enthaltenden Aminosäuren spricht man von konservativen Austauschen wenn Serin und Threonin gegeneinander ausgetauscht werden.

In gleicher Weise können auch solche Nukleotidsequenzen verwendet werden, die für Varianten der genannten Proteine kodieren, die zusätzlich am N- oder C-Terminus eine Verlängerung oder Verkürzung um mindestens eine (1) Aminosäure enthalten. Diese Verlängerung oder Verkürzung beträgt nicht mehr als 50, 40, 30, 20, 10, 5, 3 oder 2 Aminosäuren oder Aminosäurereste.

Zu den geeigneten Allelen zählen auch solche, die für Proteine kodieren, in denen mindestens eine (1) Aminosäure eingefügt (Insertion) oder entfernt (Deletion) ist. Die maximale Anzahl derartiger als Indels bezeichneter Veränderungen kann 2, 3, 5, 10, 20 in keinem Falle aber mehr als 30 Aminosäuren betreffen.

Zu den geeigneten Allelen gehören ferner solche, die durch Hybridisierung, insbesondere unter stringenten Bedingungen unter Verwendung von SEQ ID No. 3, SEQ ID No. 5 oder SEQ ID No. 7 oder Teilen davon insbesondere der Kodierregionen bzw. der dazu komplementären Sequenzen, erhältlich sind.

Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology 41: 255-260 (1991)). Die Hybridisierung findet unter stringenten Bedingungen statt, das heißt, es werden nur Hybride gebildet, bei denen Sonde und Zielsequenz, d. h. die mit der Sonde behandelten Polynukleotide, mindestens 70% identisch sind. Es ist bekannt, dass die Stringenz der Hybridisierung einschließlich der Waschschritte durch Variieren der Pufferzusammensetzung, der Temperatur und der Salzkonzentration beeinflusst bzw. bestimmt wird. Die Hybridisierungsreaktion wird im Allgemeinen bei relativ niedriger Stringenz im Vergleich zu den Waschschritten durchgeführt (Hybaid Hybridisation Guide, Hybaid Limited, Teddington, UK, 1996).

Für die Hybridisierungsreaktion kann beispielsweise ein Puffer entsprechend 5x SSC-Puffer bei einer Temperatur von ca. 50°C - 68°C eingesetzt werden. Dabei können Sonden auch mit Polynukleotiden hybridisieren, die weniger als 70% Identität zur Sequenz der Sonde aufweisen. Solche Hybride sind weniger stabil und werden durch Waschen unter stringenten Bedingungen entfernt. Dies kann beispielsweise durch Senken der Salzkonzentration auf 2x SSC und gegebenenfalls nachfolgend 0,5x SSC (The DIG System User's Guide for Filter Hybridisation, Boehringer Mannheim, Mannheim, Deutschland, 1995) erreicht werden, wobei eine Temperatur von ca. 50°C - 68°C, ca. 52°C - 68°C, ca. 54°C - 68°C, ca. 56°C - 68°C, ca. 58°C - 68°C, ca. 60°C - 68°C, ca. 62°C - 68°C, ca. 64°C - 68°C, ca. 66°C - 68°C eingestellt wird. Es ist gegebenenfalls möglich die Salzkonzentration bis auf eine Konzentration entsprechend 0,2x SSC oder 0,1x SSC zu senken. Durch schrittweise Erhöhung der Hybridisierungstemperatur in Schritten von ca. 1 - 2°C von 50°C auf 68°C können Polynukleotidfragmente isoliert werden, die beispielsweise mindestens 70% oder mindestens 80% oder mindestens 90%, mindestens 91%, mindestens 92%, mindestens 93%, mindestens 94%, mindestens 95%, mindestens 96%, mindestens 97%, mindestens 98% oder mindestens 99% Identität zur Sequenz der eingesetzten Sonde besitzen. Weitere Anleitungen zur Hybridisierung sind in Form sogenannter Kits am Markt erhältlich (z.B. DIG Easy Hyb von der Firma Roche Diagnostics GmbH, Mannheim, Deutschland, Catalog No. 1603558). Die so erhaltenen Nukleotidsequenzen kodieren für Polypeptide, die mindestens 90% Identität zu den in SEQ ID No. 4, SEQ ID No. 6 oder SEQ ID No. 8 dargestellten Aminosäuresequenzen besitzen.

Zur Erzielung einer Verstärkung können beispielsweise die Expression der Gene oder Allele oder die katalytischen Eigenschaften des Proteins erhöht werden. Gegebenenfalls können beide Maßnahmen kombiniert werden.

Zur Erzielung einer Überexpression kann beispielsweise die Kopienzahl der entsprechenden Gene erhöht werden, oder es kann die Promotor- und Regulationsregion oder die Ribosomenbindungsstelle, die sich stromaufwärts des Strukturgens befindet, mutiert werden. In gleicher Weise wirken Expressionskassetten oder Promotoren, die stromaufwärts des Strukturgens eingebaut werden. Durch den Einbau induzierbarer Promotoren ist es zusätzlich möglich, die Expression im Verlaufe der fermentativen L-Threonin-Produktion zu steigern. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Die Gene oder Genkonstrukte können entweder in Plasmiden mit unterschiedlicher Kopienzahl vorliegen oder im Chromosom integriert und amplifiziert sein. Alternativ kann weiterhin eine Überexpression der betreffenden Gene durch Veränderung der Medienzusammensetzung und Kulturführung erreicht werden.

Methoden der Überexpression sind im Stand der Technik hinlänglich - beispielsweise bei Makrides et al. (Microbiological Reviews 60 (3), 512-538 (1996)) - beschrieben. Durch Verwendung von Vektoren wird die Kopienzahl um mindestens eine (1) Kopie erhöht. Als Vektoren können Plasmide wie beispielsweise in der US 5,538,873 beschrieben verwendet werden. Als Vektoren können ebenfalls Phagen, beispielsweise der Phage Mu, wie in der EP 0 332 448 beschrieben, oder der Phage lambda (λ) verwendet werden. Eine Erhöhung der Kopienzahl kann auch dadurch erzielt werden, dass eine weitere Kopie in eine weitere Stelle des Chromosoms - beispielsweise in die attsite des Phagen λ (Yu und Court, Gene 223, 77-81 (1998)) - eingebaut wird. In der US 5,939,307 wird beschrieben, dass durch Einbau von Expressionskassetten oder Promotoren wie beispielsweise tac-Promotor, trp-Promotor, lpp-Promotor oder P_{L}-Promotor und P_{R}-Promotor des Phagen λ beispielsweise stromaufwärts des chromosomalen Threoninoperons eine Erhöhung der Expression erzielt werden konnte. In gleicher Weise können die Promotoren des Phagen T7, die gear-box-Promotoren oder der nar-Promotor verwendet werden. Derartige Expressionskassetten oder Promotoren können auch verwendet werden um, wie in der EP 0 593 792 beschrieben, plasmidgebundene Gene zu überexprimieren. Durch Verwendung des lacI^{Q}-Allels lässt sich wiederum die Expression plasmidgebundener Gene kontrollieren (Glascock und Weickert, Gene 223, 221-231 (1998)).

Anleitungen hierzu findet der Fachmann unter anderem bei Chang und Cohen (Journal of Bacteriology 134: 1141-1156 (1978)), bei Hartley und Gregori (Gene 13: 347-353 (1981)), bei Amann und Brosius (Gene 40: 183-190 (1985)), bei de Broer et al. (Proceedings of the National Academy of Sciences of the United States of America 80: 21-25 (1983)), bei LaVallie et al. (BIO/TECHNOLOGY 11: 187-193 (1993)), in PCT/US97/13359, bei Llosa et al. (Plasmid 26: 222-224 (1991)), bei Quandt und Klipp (Gene 80: 161-169 (1989)), bei Hamilton et al. (Journal of Bacteriology 171: 4617-4622 (1989)), bei Jensen und Hammer (Biotechnology and Bioengineering 58: 191-195 (1998)) und in bekannten Lehrbüchern der Genetik und Molekularbiologie.

In Enterobacteriaceae replizierbare Plasmidvektoren wie z.B. von pACYC184 abgeleitete Kloniervektoren (Bartolomé et al.; Gene 102: 75-78 (1991)), pTrc99A (Amann et al.; Gene 69: 301-315 (1988)) oder pSC101-Derivate (Vocke und Bastia; Proceedings of the National Academy of Sciences USA 80(21): 6557-6561 (1983)) können verwendet werden. In einem erfindungsgemäßen Verfahren kann man einen mit einem Plasmidvektor transformierten Stamm einsetzen, wobei der Plasmidvektor mindestens ein lamB-Gen oder Allel, oder für dessen Genprodukt LamB kodierende Nukleotidsequenzen trägt.

Unter dem Begriff Transformation versteht man die Aufnahme einer isolierten Nukleinsäure durch einen Wirt (Mikroorganismus).

Es ist ebenfalls möglich, Mutationen, welche die Expression der jeweiligen Gene betreffen, durch Sequenzaustausch (Hamilton et al.; Journal of Bacteriology 171: 4617-4622 (1989)), Konjugation oder Transduktion in verschiedene Stämme zu überführen.

Nähere Erläuterungen zu den Begriffen der Genetik und Molekularbiologie findet man in bekannten Lehrbüchern der Genetik und Molekularbiologie wie beispielsweise dem Lehrbuch von Birge (Bacterial and Bacteriophage Genetics, 4th ed., Springer Verlag, New York (USA), 2000) oder dem Lehrbuch von Berg, Tymoczko and Stryer (Biochemistry, 5th ed., Freeman and Company, New York (USA), 2002) oder dem Handbuch von Sambrook et al. (Molekular Cloning, A Laboratory Manual, (3-Volume Set), Cold Spring Harbor Laboratory Press, Cold Spring Harbor (USA), 2001).

Weiterhin kann es für die Produktion von L-Aminosäuren, insbesondere L-Threonin mit Stämmen der Familie Enterobacteriaceae vorteilhaft sein, zusätzlich zur Verstärkung des lamB-Gens, ein oder mehrere Enzyme des bekannten Threonin-Biosyntheseweges oder Enzyme des anaplerotischen Stoffwechsels oder Enzyme für die Produktion von reduziertem Nicotinamid-Adenin-Dinukleotid-Phosphat oder Enzyme der Glykolyse oder PTS-Enzyme oder Enzyme des Schwefelstoffwechsels zu verstärken. Die Verwendung endogener Gene wird im Allgemeinen bevorzugt.

So können beispielsweise gleichzeitig eines oder mehrere der Gene, ausgewählt aus der Gruppe
- •: mindestens ein Gen des für die Aspartatkinase, die Homoserin-Dehydrogenase, die Homoserinkinase und die Threoninsynthase kodierenden thrABC-Operons (US-A- 4,278,765),
- •: das für die Pyruvat-Carboxylase kodierende pyc-Gen von Corynebacterium glutamicum (WO 99/18228),
- •: das für die Phosphoenolpyruvat-Synthase kodierende pps- Gen (Molecular and General Genetics 231(2): 332-336 (1992)),
- •: das für die Phosphoenolpyruvat-Carboxylase kodierende ppc-Gen (WO 02/064808),
- •: die für die Untereinheiten der Pyridin-Transhydrogenase kodierenden Gene pntA und pntB (European Journal of Biochemistry 158: 647-653 (1986)),
- •: das für das Homoserinresistenz vermittelnde Protein kodierende Gen rhtB (EP-A-0 994 190),
- •: das für das Threoninresistenz vermittelnde Protein kodierende Gen rhtC (EP-A-1 013 765),
- •: das für das Threonin-Export-Carrier-Protein kodierende thrE-Gen von Corynebacterium glutamicum (WO 01/92545),
- •: das für die Glutamat-Dehydrogenase kodierende gdhA-Gen (Nucleic Acids Research 11: 5257-5266 (1983); Gene 23: 199-209 (1983)),
- •: das für die Phosphoglucomutase kodierende pgm-Gen (WO 03/004598),
- •: das für die Fructose Biphosphat Aldolase kodierende fba- Gen (WO 03/004664),
- •: das für die Phosphohistidin-Protein-Hexose- Phosphotransferase des Phosphotransferase-Systems PTS kodierende ptsH-Gen des ptsHIcrr-Operons (WO 03/004674),
- •: das für das Enzym I des Phosphotransferase-Systems PTS kodierende ptsI-Gen des ptsHIcrr-Operons (WO 03/004674),
- •: das für die Glucose-spezifische IIA Komponente des Phosphotransferase-Systems PTS kodierende crr-Gen des ptsHIcrr-Operons (WO 03/004674),
- •: das für die Glucose-spezifische IIBC Komponente kodierende ptsG-Gen (WO 03/004670),
- •: das für den Regulator des Leucin-Regulons kodierende lrp-Gen (WO 03/004665),
- •: das für den Regulator des fad-Regulons kodierende fadR- Gen (WO 03/038106),
- •: das für den Regulator des zentralen Intermediärstoffwechsels kodierende iclR-Gen (WO 03/038106),
- •: das für die kleine Untereinheit der Alkyl Hydroperoxid Reduktase kodierende ahpC-Gen des ahpCF-Operons (WO 03/004663),
- •: das für die große Untereinheit der Alkyl Hydroperoxid Reduktase kodierende ahpF-Gen des ahpCF-Operons (WO 03/004663),
- •: das für die Cystein-Synthase A kodierende cysK-Gen (WO 03/006666),
- •: das für den Regulator des cys-Regulons kodierende cysB- Gen (WO 03/006666),
- •: das für das Flavoprotein der NADPH-Sulfit-Reduktase kodierende cysJ-Gen des cysJIH-Operons (WO 03/006666),
- •: das für das Hämoprotein der NADPH-Sulfit-Reduktase kodierende cysI-Gen des cysJIH-Operons (WO 03/006666),
- •: das für die Adenylylsulfat-Reduktase kodierende cysH-Gen des cysJIH-Operons (WO 03/006666),
- •: das für ein Membranprotein mit anti-sigmaE-Aktivität kodierende rseA-Gen des rseABC-Operons (WO 03/008612), • das für einen globalen Regulator des sigmaE-Faktors kodierende rseC-Gen des rseABC-Operons (WO 03/008612),
- •: das für die Decarboxylase Untereinheit der 2- Ketoglutarat Dehydrogenase kodierende sucA-Gen des sucABCD-Operons (WO 03/008614),
- •: das für die Dihydrolipoyltranssuccinase E2 Untereinheit der 2-Ketoglutarat Dehydrogenase kodierende sucB-Gen des sucABCD-Operons (WO 03/008614),
- •: das für die β-Untereinheit der Succinyl-CoA Synthetase kodierende sucC-Gen des suc ABCD-Operons (WO 03/008615),
- •: das für die α-Untereinheit der Succinyl-CoA Synthetase kodierende sucD-Gen des sucABCD-Operons (WO 03/008615),
- •: das für die E1-Komponente des Pyruvat-Dehydrogenase- Komplexes kodierende aceE-Gen (WO 03/076635),
- •: das für die E2-Komponente des Pyruvat-Dehydrogenase- Komplexes kodierende aceF-Gen (WO 03/076635),
- •: das für den Regulator der SigmaE-Faktor-Aktivität kodierende rseB-Gen (Molecular Microbiology 24(2): 355- 371 (1997)),
- •: das für den positiven transkriptionalen Regulator des Maltose Regulons kodierende malT-Gen (Gene 42: 201-208 (1986), DE102004003411.7),
- •: das Genprodukt des offenen Leserahmens (ORF) yaaU von Escherichia coli (Accession Number AE000114 des National Center for Biotechnology Information (NCBI, Bethesda, MD, USA), DE10361268.8),
- •: das Genprodukt des offenen Leserahmens (ORF) yodA von Escherichia coli (Accession Number AE000288 des National Center for Biotechnology Information (NCBI, Bethesda, MD, USA), DE10361192.4),
- •: das Genprodukt des offenen Leserahmens (ORF) yibD von Escherichia coli (Accession Number AE000439 des National Center for Biotechnology Information (NCBI, Bethesda, MD, USA), DE102004005836.9)

verstärkt, insbesondere überexprimiert werden. Weiterhin kann es für die Produktion von L-Aminosäuren, insbesondere L-Threonin vorteilhaft sein, zusätzlich zur Verstärkung des lamB-Gens, eines oder mehrere der Gene ausgewählt aus der Gruppe
- •: das für die Threonin-Dehydrogenase kodierende tdh-Gen (Journal of Bacteriology 169: 4716-4721 (1987)),
- •: das für die Malat-Dehydrogenase (E.C. 1.1.1.37) kodierende mdh-Gen (Archives in Microbiology 149: 36-42 (1987)),
- •: das Genprodukt des offenen Leserahmens (ORF) yjfA von Escherichia coli (Accession Number AAC77180 des National Center for Biotechnology Information (NCBI, Bethesda, MD, USA), WO 02/29080),
- •: das Genprodukt des offenen Leserahmens (ORF) ytfP von Escherichia coli (Accession Number AAC77179 des National Center for Biotechnology Information (NCBI, Bethesda, MD, USA), WO 02/29080),
- •: das für das Enzym Phosphoenolpyruvat-Carboxykinase kodierende pckA-Gen (WO 02/29080),
- •: das für die Pyruvat-Oxidase kodierende poxB-Gen (WO 02/36797),
- •: das für den DgsA-Regulator des Phosphotransferase- Systems kodierende dgsA-Gen (WO 02/081721), das auch unter der Bezeichnung mlc-Gen bekannt ist,
- •: das für den Fructose-Repressor kodierende fruR-Gen (WO 02/081698), das auch unter der Bezeichnung cra-Gen bekannt ist,
- •: das für den Sigma³⁸-Faktor kodierende rpoS-Gen (WO 01/05939), das auch unter der Bezeichnung katF-Gen bekannt ist und
- •: das für die Aspartat Ammonium-Lyase kodierende aspA-Gen (WO 03/008603)
abzuschwächen, insbesondere auszuschalten oder die Expression zu verringern.

Der Begriff "Abschwächung" beschreibt in diesem Zusammenhang die Verringerung oder Ausschaltung der intrazellulären Aktivität oder Konzentration eines oder mehrerer Enzyme bzw. Proteine in einem Mikroorganismus, die durch die entsprechende DNA kodiert werden, indem man beispielsweise einen schwächeren Promotor als im Ausgangsstamm oder ein Gen bzw. Allel verwendet, das für ein entsprechendes Enzym bzw. Protein mit einer niedrigen Aktivität kodiert bzw. das entsprechende Enzym bzw. Protein oder das Gen inaktiviert und gegebenenfalls diese Maßnahmen kombiniert.

Durch die Maßnahmen der Abschwächung wird die Aktivität oder Konzentration des entsprechenden Proteins im allgemeinen auf 0 bis 75%, 0 bis 50%, 0 bis 25%, 0 bis 10% oder 0 bis 5% der Aktivität oder Konzentration des Wildtyp-Proteins, beziehungsweise der Aktivität oder Konzentration des Proteins im nicht rekombinanten Ausganges-Mikroorganismus, herabgesenkt. Als Ausgangs-Mikroorganismus oder Elternstamm (parent strain) wird der Mikroorganismus verstanden, an dem die erfinderischen Maßnahmen durchgeführt werden.

Zur Erzielung einer Abschwächung können beispielsweise die Expression der Gene oder offenen Leserahmen oder die katalytischen Eigenschaften der Enzymproteine herabgesetzt bzw. ausgeschaltet werden. Gegebenenfalls können beide Maßnahmen kombiniert werden.

Die Verringerung der Genexpression kann durch geeignete Kulturführung, durch genetische Veränderung (Mutation) der Signalstrukturen der Genexpression oder auch durch Antisense-RNA Technik erfolgen. Signalstrukturen der Genexpression sind beispielsweise Repressorgene, Aktivatorgene, Operatoren, Promotoren, Attenuatoren, Ribosomenbindungsstellen, das Startkodon und Terminatoren. Angaben hierzu findet der Fachmann unter anderem beispielsweise bei Jensen und Hammer (Biotechnology and Bioengineering 58: 191-195 (1998)), bei Carrier und Keasling (Biotechnology Progress 15: 58-64 (1999)), Franch und Gerdes (Current Opinion in Microbiology 3: 159-164 (2000)) und in bekannten Lehrbüchern der Genetik und Molekularbiologie wie beispielsweise dem Lehrbuch von Knippers ("Molekulare Genetik", 6. Auflage, Georg Thieme Verlag, Stuttgart, Deutschland, 1995) oder dem von Winnacker ("Gene und Klone", VCH Verlagsgesellschaft, Weinheim, Deutschland, 1990).

Mutationen, die zu einer Veränderung beziehungsweise Herabsetzung der katalytischen Eigenschaften von Enzymproteinen führen, sind aus dem Stand der Technik bekannt. Als Beispiele seien die Arbeiten von Qiu und Goodman (Journal of Biological Chemistry 272: 8611-8617 (1997)), Yano et al. (Proceedings of the National Academy of Sciences of the United States of America 95: 5511-5515 (1998)), Wente und Schachmann (Journal of Biological Chemistry 266: 20833-20839 (1991)) genannt.

Zusammenfassende Darstellungen können bekannten Lehrbüchern der Genetik und Molekularbiologie wie z.B. dem von Hagemann ("Allgemeine Genetik", Gustav Fischer Verlag, Stuttgart, 1986) entnommen werden.

Als Mutationen kommen Transitionen, Transversionen, Insertionen und Deletionen von mindestens einem (1) Basenpaar bzw. Nukleotid in Betracht. In Abhängigkeit von der Wirkung des durch die Mutation hervorgerufenen Aminosäureaustausches auf die Enzymaktivität wird von Fehlsinnmutationen ("missense mutations") oder Nichtsinnmutationen ("nonsense mutations") gesprochen. Die Fehlsinnmutation führt zu einem Austausch einer gegebenen Aminosäure in einem Protein gegen eine andere, wobei es sich insbesondere um einen nicht-konservative Aminosäureaustausch handelt. Hierdurch wird die Funktionsfähigkeit bzw. Aktivität des Proteins beeinträchtigt und auf einen Wert von 0 bis 75%, 0 bis 50%, 0 bis 25%, 0 bis 10% oder 0 bis 5% reduziert. Die Nichtsinnmutation führt zu einem Stop-Kodon im Kodierbereich des Gens und damit zu einem vorzeitigen Abbruch der Translation. Insertionen oder Deletionen von mindestens einem Basenpaar in einem Gen führen zu Rasterverschiebungsmutationen ("frame shift mutations"), die dazu führen, dass falsche Aminosäuren eingebaut werden oder die Translation vorzeitig abbricht. Entsteht als Folge der Mutation ein Stop-Kodon im Kodierbereich, so führt dies ebenfalls zu einem vorzeitigen Abbruch der Translation. Deletionen von mindestens einem (1) oder mehreren Kodonen führen typischerweise ebenfalls zu einem vollständigen Ausfall der Enzymaktivität.

Anleitungen zur Erzeugung derartiger Mutationen gehören zum Stand der Technik und können bekannten Lehrbüchern der Genetik und Molekularbiologie wie z.B. dem Lehrbuch von Knippers ("Molekulare Genetik", 6. Auflage, Georg Thieme Verlag, Stuttgart, Deutschland, 1995), dem von Winnacker ("Gene und Klone", VCH Verlagsgesellschaft, weinheim, Deutschland, 1990) oder dem von Hagemann ("Allgemeine Genetik", Gustav Fischer Verlag, Stuttgart, 1986) entnommen werden.

Geeignete Mutationen in den Genen können durch Gen- bzw. Allelaustausch in geeignete Stämme eingebaut werden.

Eine gebräuchliche Methode ist die von Hamilton et al. (Journal of Bacteriology 171: 4617-4622 (1989)) beschriebene Methode des Genaustauschs mit Hilfe eines konditional replizierenden pSC101-Derivates pMAK705. Andere im Stand der Technik beschriebene Methoden wie beispielsweise die von Martinez-Morales et al. (Journal of Bacteriology 181: 7143-7148 (1999)) oder die von Boyd et al. (Journal of Bacteriology 182:842-847 (2000)) können gleichfalls benutzt werden.

Es ist ebenfalls möglich, Mutationen in den jeweiligen Genen oder Mutationen, die die Expression der jeweiligen Gene oder offenen Leserahmen betreffen, durch Konjugation oder Transduktion in verschiedene Stämme zu überführen.

Weiterhin kann es für die Produktion von L-Aminosäuren, insbesondere L-Threonin vorteilhaft sein, zusätzlich zur

Verstärkung des lamB-Gens, unerwünschte Nebenreaktionen auszuschalten (Nakayama: "Breeding of Amino Acid Producing Microorganisms", in: Overproduction of Microbial Products, Krumphanzl, Sikyta, Vanek (eds.), Academic Press, London, UK, 1982).

Die erfindungsgemäß hergestellten Mikroorganismen können im batch - Verfahren (Satzkultivierung), im fed batch (Zulaufverfahren), im repeated fed batch-Verfahren (repetitives zulaufverfahren) oder in einem kontinuierlichen Verfahren (US .........) kultiviert werden. Eine Zusammenfassung über bekannte Kultivierungsmethoden sind im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/wiesbaden, 1994)) beschrieben.

Das zu verwendende Kulturmedium muss in geeigneter Weise den Ansprüchen der jeweiligen Stämme genügen.

Beschreibungen von Kulturmedien verschiedener Mikroorganismen sind im Handbuch "Manual of Methods for General Bacteriology" der American Society for Bacteriology (Washington D.C., USA, 1981) enthalten.

Als Kohlenstoffquelle können Zucker und Kohlehydrate wie z.B. Glucose, Saccharose, Lactose, Fructose, Maltose, Melasse, Stärke und gegebenenfalls Cellulose, Öle und Fette wie z.B. Sojaöl, Sonnenblumenöl, Erdnussöl und Kokosfett, Fettsäuren wie z.B. Palmitinsäure, Stearinsäure und Linolsäure, Alkohole wie z.B. Glycerin und Ethanol und organische Säuren wie z.B. Essigsäure verwendet werden. Diese Stoffe können einzeln oder als Mischung verwendet werden.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium-haltigen Salze verwendet werden. Das Kulturmedium muss weiterhin Salze von Metallen enthalten, wie z.B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter weise während der Kultivierung zugefüttert werden.

Die Fermentation wird im allgemeinen bei einem pH-Wert von 5,5 bis 9,0, insbesondere 6,0 bis 8,0, durchgeführt. Zur pH-Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z.B. Fettsäurepolyglykolester eingesetzt werden. Zur Aufrechterhaltung der Stabilität von Plasmiden können dem Medium geeignete selektiv wirkende Stoffe z.B. Antibiotika hinzugefügt werden. Um aerobe Bedingungen aufrechtzuerhalten, werden Sauerstoff oder Sauerstoffhaltige Gasmischungen wie z.B. Luft in die Kultur eingetragen. Die Temperatur der Kultur liegt normalerweise bei 25°C bis 45°C und vorzugsweise bei 30°C bis 40°C. Die Kultur wird solange fortgesetzt, bis sich ein Maximum an L-Aminosäuren bzw. L-Threonin gebildet hat. Dieses Ziel wird normalerweise innerhalb von 10 Stunden bis 160 Stunden erreicht.

Die Analyse von L-Aminosäuren kann durch Anionenaustauschchromatographie mit anschließender Ninhydrin Derivatisierung erfolgen, so wie bei Spackman et al. (Analytical Chemistry 30: 1190-1206 (1958)) beschrieben, oder sie kann durch reversed phase HPLC erfolgen, so wie bei Lindroth et al. (Analytical Chemistry 51: 1167-1174 (1979)) beschrieben.

Das erfindungsgemäße Verfahren dient zur fermentativen Herstellung von L-Aminosäuren, wie beispielsweise L-Threonin, L-Isoleucin, L-Valin, L-Methionin, L-Homoserin, L-Tryptophan und L-Lysin, insbesondere L-Threonin.

Die vorliegende Erfindung wird im Folgenden anhand von Ausführungsbeispielen näher erläutert.

Verwendete Minimal- (M9) und Vollmedien (LB) für Escherichia coli sind von J.H. Miller (A Short Course in Bacterial Genetics (1992), Cold Spring Harbor Laboratory Press) beschrieben. Die Isolierung von Plasmid-DNA aus Escherichia coli sowie alle Techniken zur Restriktion, Ligation, Klenow- und alkalische Phosphatasebehandlung werden nach Sambrook et al. (Molecular Cloning - A Laboratory Manual (1989) Cold Spring Harbor Laboratory Press) durchgeführt. Die Transformation von Escherichia coli wird, wenn nicht anders beschrieben, nach Chung et al. (Proceedings of the National Academy of Sciences of the United States of America 86: 2172-2175 (1989)) durchgeführt.

Die Bebrütungstemperatur bei der Herstellung von Stämmen und Transformanten ist 37°C.

### Beispiel 1

Konstruktion des Expressionsplasmides pTrc99AlamB

Das lamB-Gen aus E. coli K12 wird unter Anwendung der Polymerase-Kettenreaktion (PCR) sowie synthetischen Oligonukleotiden amplifiziert. Ausgehend von der Nukleotidsequenz des lamB-Gens in E. coli K12 MG1655 (Accession Number AE000477), Blattner et al. (Science 277: 1453-1474 (1997)) werden PCR-Primer synthetisiert (MWG Biotech, Ebersberg, Deutschland). Die Sequenzen der Primer werden so modifiziert, dass Erkennungsstellen für Restriktionsenzyme entstehen. Für den P_lam1neu-Primer wird die Erkennungssequenz für XbaI und für den P_lam2neu-Primer die Erkennungssequenz für HindIII gewählt, die in der unten dargestellten Nukleotidabfolge durch Unterstreichen markiert sind:
p_lam1neu: 5' -TCTAGAGCCTGTCACAGGTGATGTGAA- 3' (SEQ ID No. 1)
P_lam2neu: 5' -AAGCTTACAGCCGTTGTAGGCCTGATA- 3' (SEQ ID No. 2)

Die für die PCR eingesetzte chromosomale E. coli K12 MG1655 DNA wird nach Herstellerangaben mit "Qiagen Genomic-tips 100/G" (QIAGEN, Hilden, Deutschland) isoliert. Ein 1498 bp großes DNA-Fragment kann mit den spezifischen Primern unter Standard-PCR-Bedingungen (Innis et al. (1990) PCR Protocols. A Guide to Methods and Applications, Academic Press) mit der Vent-DNA-Polymerase (New England Biolaps GmbH, Frankfurt, Deutschland) amplifiziert werden (SEQ ID No. 3).

Das amplifizierte lamB-Fragment wird mit dem Vektor pCR-Blunt II-TOPO (Zero TOPO TA Cloning Kit, Invitrogen, Groningen, Niederlande) den Herstellerangaben entsprechend ligiert und in den E. coli Stamm TOP10 transformiert. Die Selektion Plasmid tragender Zellen erfolgt auf LB Agar, der mit 50 µg/ml Kanamycin versetzt ist. Nach der Plasmid DNA Isolierung wird der Vektor mit den Enzymen EcoRI und EcoRV gespalten und nach Überprüfung der Spaltung im 0,8%igen Agarosegel mit pCRBluntlamB bezeichnet.

Die Nukleotidsequenz des amplifizierten DNA-Fragmentes bzw. PCR-Produktes wird nach der Dideoxy-Kettenabbruch-Methode von Sanger et al. (Proceedings of the National Academy of Sciences U.S.A., 74:5463-5467 (1977)) mit dem "ABI Prism 377" Sequenziergerät der Firma PE Applied Biosystems (Weiterstadt, Deutschland) überprüft. Die Sequenz des PCR-Produktes entspricht den Positionen 1-1498 der in der SEQ ID No. 3 dargestellten Sequenz. Die Aminosäuresequenz des dazugehörigen LamB-Proteins ist in der SEQ ID No. 4 dargestellt.

Anschließend wird der Vektor pCRBluntlamB mit den Enzymen HindIII und XbaI gespalten und das ca. 1500bp lamB-Fragment nach Auftrennung im 0,8%igem Agarosegel aus dem Gel isoliert (QIAquick Gel Extraction Kit, QIAGEN, Hilden, Deutschland) und mit dem Vektor pTrc99A (Pharmacia Biotech, Uppsala, Schweden), der mit den Enzymen HindIII und XbaI verdaut worden ist, ligiert. Der E. coli Stamm DH5α (Grant et al.; Proceedings of the National Academy of Sciences USA, 87 (1990) 4645-4649) wird mit dem Ligationsansatz transformiert und Plasmid tragende Zellen auf LB Agar, der mit 50 µg/ml Ampicillin versetzt ist, selektioniert.

Die erfolgreiche Klonierung kann nach der Plasmid DNA Isolierung durch die Kontrollspaltung mit den Enzymen EcoRV bzw. SspI nachgewiesen werden.

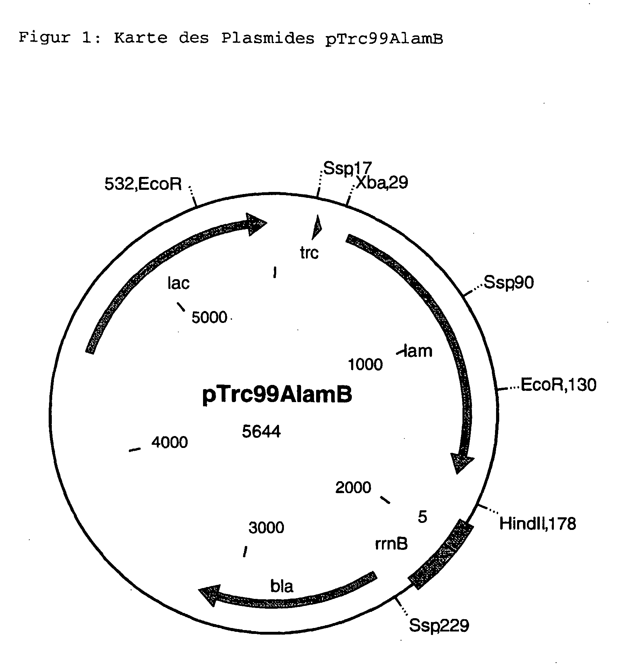

Das Plasmid wird als pTrc99AlamB (Figur 1) bezeichnet.

### Beispiel 2

### Konstruktion des Expressionsplasmides pMW2181amB

Der in Beispiel 1 beschriebene Vektor pCRBluntlamB wird mit den Enzymen HindIII und XbaI gespalten und das lamB-Fragment nach Auftrennung im 0,8%igem Agarosegel aus dem Gel isoliert (QIAquick Gel Extraction Kit, QIAGEN, Hilden, Deutschland) und mit dem low-copy Vektor pMW218 (Nippon Gene, Toyama, Japan), der mit den Enzymen HindIII und XbaI verdaut worden ist, ligiert. Der E. coli Stamm DH5α (Grant et al.; Proceedings of the National Academy of Sciences USA, 87 (1990) 4645-4649) wird mit dem Ligationsansatz transformiert und Plasmid tragende Zellen auf LB Agar, der mit 50 µg/ml Kanamycin versetzt ist, selektioniert.

Die erfolgreiche Klonierung kann nach der Plasmid DNA Isolierung durch die Kontrollspaltung mit dem Enzym ClaI nachgewiesen werden.

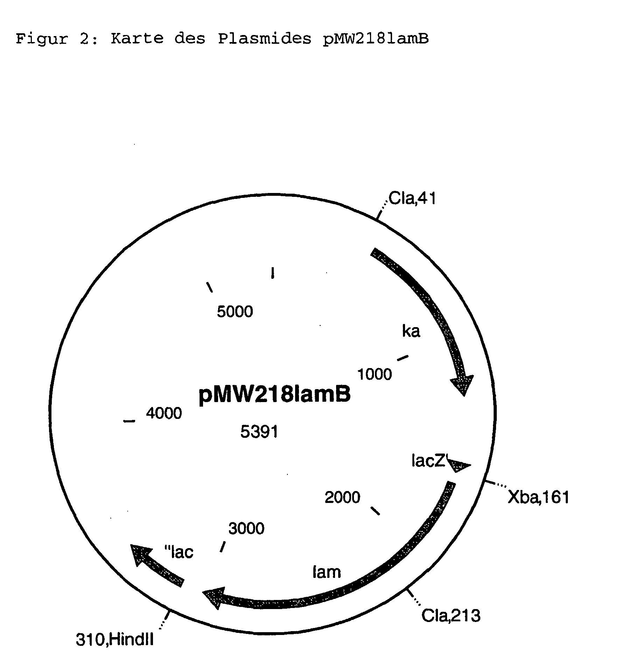

Das Plasmid wird als pMW2181amB (Figur 2) bezeichnet.

### Beispiel 3

Herstellung von L-Threonin mit den Stämmen MG442/pTrc99AlamB und MG442/pMW2181amB

Der L-Threonin produzierende E. coli Stamm MG442 ist in der Patentschrift US-A- 4,278,765 beschrieben und bei der Russischen Nationalsammlung für industrielle Mikroorganismen (VKPM, Moskau, Russland) als CMIM B-1628 hinterlegt.

Der Stamm MG442 wird mit den in Beispiel 1 und 2 beschriebenen Expressionsplasmiden pTrc99AlamB und pMW2181amB und mit den Vektoren pTrc99A und pMW218 transformiert und auf LB Agar mit 50 µg/ml Ampicillin bzw. 50 µg/ml Kanamycin Plasmid tragende Zellen selektioniert.

Auf diese Weise entstehen die Stämme MG442/pTrc99AlamB, MG442/pTrc99A, MG442/pMW2181amB und MG442/pMW218. Ausgewählte Einzelkolonien werden anschließend auf Minimalmedium mit der folgenden Zusammensetzung: 3,5 g/l Na₂HPO₄*2H₂O, 1,5 g/l KH₂PO₄ 1 g/l NH₄Cl, 0,1 g/l MgSO₄*7H₂O, 2 g/l Glucose, 20 g/l Agar, 50 mg/l Ampicillin bzw. 50 mg/l Kanamycin, weiter vermehrt. Die Bildung von L-Threonin wird in batch Kulturen von 10 ml, die in 100 ml Erlenmeierkolben enthalten sind, überprüft. Dazu wird 10 ml Vorkulturmedium der folgenden Zusammensetzung: 2 g/l Hefeextrakt, 10 g/l (NH₄)₂SO₄, 1 g/l KH₂PO₄, 0,5 g/l MgSO₄*7H₂O, 15 g/l CaCO₃, 20 g/l Glucose, 50 mg/l Ampicillin bzw. 50 mg/l Kanamycin, beimpft und für 16 Stunden bei 37°C und 180 rpm auf einem ESR Inkubator der Firma Kühner AG (Birsfelden, Schweiz) inkubiert. Je 250 µl dieser Vorkultur werden in 10 ml Produktionsmedium (25 g/l (NH₄)₂SO₄, 2 g/l KH₂PO₄, 1 g/l MgSO₄*7H₂O 0,03 g/l FeSO₄*7H₂O 0,018 g/l MnSO₄*1H₂O, 30 g/l CaCO₃, 20 g/l Glucose, 50 mg/l Ampicillin bzw. 50 mg/l Kanamycin) überimpft und für 48 Stunden bei 37°C inkubiert. Nach der Inkubation wird die optische Dichte (OD) der Kultursuspension mit einem LP2W-Photometer der Firma Dr. Lange (Düsseldorf, Deutschland) bei einer Messwellenlänge von 660 nm bestimmt.

Anschließend wird die Konzentration an gebildetem L-Threonin im steril filtrierten Kulturüberstand mit einem Aminosäureanalysator der Firma Eppendorf-BioTronik (Hamburg, Deutschland) durch Ionenaustauschchromatographie und Nachsäulenreaktion mit Ninhydrindetektion bestimmt.

In Tabelle 1 ist das Ergebnis des Versuches dargestellt.

**Tabelle 1**

| Stamm | OD (660 nm) | L-Threonin g/l |
|---|---|---|
| MG442/pTrc99A | 2,0 | 2,1 |
| MG442/pTrc99AlamB | 2,0 | 2,6 |
| MG442/pMW218 | 6,2 | 1,7 |
| MG442/pMW2181amB | 5,7 | 2,5 |

Kurze Beschreibung der Figuren:
Figur 1: Karte des das lamB-Gen enthaltenden Plasmides pTrc99AlamB
Figur 2: Karte des das lamB-Gen enthaltenden Plasmides pMW2181amB

Längenangaben sind als ca.-Angaben aufzufassen. Die verwendeten Abkürzungen und Bezeichnungen haben folgende Bedeutung:
- •: bla: Gen, welches für die Ampicillinresistenz kodiert
- •: kan: Gen, welches für die Kanamycinresistenz kodiert
- •: lac Iq: Gen für das Repressorprotein des trc-Promotors
- •: lacZ': Genfragment, welches für das α-Peptid der β- Galaktosidase kodiert
- •: trc: trc-Promotorregion, IPTG-induzierbar
- •: lamB: Kodierregion des lamB-Gens
- •: 5S: 5S rRNA-Region
- •: rrnBT: rRNA-Terminator-Region

Die Abkürzungen für die Restriktionsenzyme haben folgende Bedeutung:
- •: XbaI: Restriktionsendonuklease aus Xanthomonas badrii
- •: HindIII: Restriktionsendonuklease aus Haemophilus influenzae
- •: ClaI: Restriktionsendonuklease aus Caryophanon latum
- •: SspI: Restriktionsendonuklease aus Sphaerotilus species
- •: EcoRV: Restriktionsendonuklease aus Escherichia coli

## Patentansprüche

1. Rekombinante Mikroorganismen der Familie Enterobacteriaceae, die ein überexprimiertes lamB-Gen enthalten, das für ein Polypeptid mit der Aktivität des Maltoporin LamB kodiert, und die L-Aminosäuren produzieren,und im Medium anreichern, wobei die Überexpression erhalten wird durch die Transformation eines die L-Aminosäuren produzierenden Stammes mit einem Plasmid, das ds lamB-Gen trägt.

2. Mikroorganismen gemäß Anspruch 1, die zusätzlich mindestens ein Gen des für die Aspartatkinase, die Homoserin-Dehydrogenase, die Homoserinkinase und die Threoninsynthase kodierenden thrABC-Operons enthalten und L-Threonin produzieren.

3. Mikroorganismen der Familie Enterobacteriaceae gemäss den Ansprüchen 1 und 2, in denen ein Polynukleotid überexprimiert wird, das für ein Polypeptid kodiert, das zu mindestens 90 %, insbesondere zu mindestens 95 %, bevorzugt 99 % identisch ist mit einer Aminosäuresequenz, ausgewählt aus der Gruppe SEQ ID No. 4, SEQ ID No. 6, und SEQ ID No. 8, wobei das Polypeptid die Aktivität des Maltoporin LamB besitzt.

4. Mikroorganismen gemäss Anspruch 3, enthaltend ein dem lamB-Gen entsprechendes überexprimiertes Polynukleotid ausgewählt aus der Gruppe:
a) Polynukleotid mit der Nukleotidsequenz aus SEQ ID No. 3, SEQ ID No. 5 oder SEQ ID No. 7;
b) Polynukleotid mit einer Nukleotidsequenz, die der SEQ ID No. 3, SEQ ID No. 5 oder SEQ ID No. 7 im Rahmen der Degeneration des genetischen Codes entspricht;
c) Polynukleotidsequenz mit einer Sequenz, die mit der zur Sequenz SEQ ID No. 3, SEQ ID No. 5 oder SEQ ID No. 7 komplementären Sequenz unter stringenten Bedingungen hybridisiert;
d) Polynukleotid mit einer Sequenz SEQ ID No. 3, SEQ ID No. 5 oder SEQ ID No. 7, die funktionsneutrale Sinnmutanten enthält.

5. Mikroorganismen gemäss Anspruch 3, wobei das Polypeptid eine Aminosäuresequenz besitzt, die zu wenigstens 95 % identisch ist mit einer der Sequenzen ausgewählt aus der Gruppe SEQ ID No. 4, SEQ ID No. 6 und SEQ ID No. 8.

6. Mikroorganismen gemäss Anspruch 3, wobei das Polypeptid die Aminosäuresequenz aufweist, die identisch ist mit der aus SEQ ID No. 4, SEQ ID No. 6 oder SEQ ID No. 8.

7. Mikroorganismen gemäss den Ansprüchen 1 bis 6, in denen die Kopienzahl des lamB-Gens oder der Polynukleotid-Sequenzen mit dieser Funktion um mindestens 1 erhöht vorliegt.

8. Mikroorganismen gemäss Anspruch 7, wobei die Erhöhung der Kopienzahl des lamB-Gens um mindestens 1 durch Integration des Gens oder der Polynukleotid-Sequenzen mit dieser Funktion in das Chromosom der Mikroorganismen bewirkt wird.

9. Mikroorganismen gemäss Anspruch 7, wobei die Erhöhung der Kopienzahl des lamB-Gens um mindestens 1 durch einen extrachromosomal replizierenden Vektor erzielt wird.

10. Mikroorganismen gemäss den Ansprüchen 1 bis 9, bei denen durch die Verstärkung des lamB-Gens die Konzentration oder Aktivität des LamB-Genproduktes (Proteins) um mindestens 10% erhöht sind, bezogen auf die Aktivität oder Konzentration des Genproduktes im nicht rekombinanten Ausgangsstamm.

11. Mikroorganismen gemäss den Ansprüchen 1 bis 10, **dadurch gekennzeichnet, dass** die Mikroorganismen aus den Gattungen Escherichia, Erwinia, Providencia und Serratia ausgewählt sind.

12. Verfahren zur Herstellung von L-Aminosäuren durch Fermentation von rekombinanten Mikroorganismen der Familie Enterobacteriaceae, **dadurch gekennzeichnet, dass** man
a) die die gewünschte L-Aminosäure produzierenden Mikroorganismen gemäß den Ansprüchen 1 bis 11 in einem Medium kultiviert unter Bedingungen, bei denen die gewünschte L-Aminosäureim Medium angereichert wird, und
b) die gewünschte L-Aminosäure isoliert, wobei Bestandteile der Fermentationsbrühe und/oder die Biomasse in ihrer Gesamtheit oder Anteilen (≥ 0 bis 100 %) im isolierten Produkt verbleiben oder vollständig entfernt werden.

13. Verfahren gemäss Anspruch 12, **dadurch gekennzeichnet, dass** man zur Herstellung von L-Threonin Mikroorganismen der Familie Enterobacteriaceae fermentiert, in denen man zusätzlich gleichzeitig eines oder mehrere der Gene, ausgewählt aus der Gruppe:
a) mindestens ein Gen des für die Aspartatkinase, die Homoserin-Dehydrogenase, die Homoserinkinase und die Threoninsynthase kodierenden thrABC-Operons,
b) das für die Pyruvat-Carboxylase kodierende pyc-Gen von Corynebacterium glutamicum,
c) das für die Phosphoenolpyruvat-Synthase kodierende pps-Gen,
d) das für die Phosphoenolpyruvat-Carboxylase kodierende ppc-Gen,
e) die für die Untereinheiten der Pryridin-Transhydrogenase kodierenden Gene pntA und pntB,
f) das für das Homoserinresistenz vermittelnde Protein kodierende Gen rhtB,
g) das für das Threoninresistenz vermittelnde Protein kodierende Gen rhtC,
h) das für das Threonin-Export-Carrier-Protein kodierende thrE-Gen aus Corynebacterium glutamicum,
i) das für die Glutamat-Dehydrogenase kodierende gdhA-Gen,
k) das für die Phosphoglucomutase kodierende pgm-Gen,
l) das für die Fructose Biphosphat Aldolase kodierende fba-Gen,
m) das für die Phosphohistidin-Protein-Hexose-Phosphotransferase kodierende ptsH-Gen,
n) das für das Enzym I des Phosphotransferase-Systems kodierende ptsI-Gen,
o) das für die Glucose-spezifische IIA Komponente kodierende crr-Gen,
p) das für die Glucose-spezifische IIBC Komponente kodierende ptsG-Gen,
q) das für den Regulator des Leucin-Regulons kodierende lrp-Gen,
r) das für den Regulator des fad-Regulons kodierende fadR-Gen,
s) das für den Regulator des zentralen Intermediärstoffwechsels kodierende iclR-Gen,
t) das für die kleine Untereinheit der Alkyl Hydroperoxid Reduktase kodierende ahpC-Gen,
u) das für die große Untereinheit der Alkyl Hydroperoxid Reduktase kodierende ahpF-Gen,
v) das für die Cystein-Synthase A kodierende cysK-Gen,
w) das für den Regulator des cys-Regulons kodierende cysB-Gen,
x) das für das Flavoprotein der NADPH-Sulfit-Reduktase kodierende cysJ-Gen,
y) das für das Hämoprotein der NADPH-Sulfit-Reduktase kodierende cysI-Gen,
z) das für die Adenylylsulfat-Reduktase kodierende cysH-Gen,
z1) das für ein Membranprotein mit anti-sigmaE-Aktivität kodierende rseA-Gen,
z2) das für einen globalen Regulator des sigmaE-Faktors kodierende rseC-Gen
z3) das für die Decarboxylase Untereinheit der 2-Ketoglutarat Dehydrogenase kodierende sucA-Gen,
z4) das für die Dihydrolipoyltranssuccinase E2 Untereinheit der 2-Ketoglutarat Dehydrogenase kodierende sucB-Gen,
z5) das für die β-Untereinheit der Succinyl-CoA Synthetase kodierende sucC-Gen,
z6) das für die α-Untereinheit der Succinyl-CoA Synthetase kodierende sucD-Gen,
z7) das für die E1-Komponente des Pyruvat-Dehydrogenase-Komplexes kodierende aceE-Gen,
z8) das für die E2-Komponente des Pyruvat-Dehydrogenase-Komplexes kodierende aceF-Gen,
z9) das für den Regulator der SigmaE-Faktor-Aktivität kodierende rseB-Gen,
z10) das für den positiven transkriptionalen Regulator des Maltose Regulons kodierende malT-Gen,
z11) das Genprodukt des offenen Leserahmens (ORF) yaaU von Escherichia coli,
z12) das Genprodukt des offenen Leserahmens (ORF) yodA von Escherichia coli und
z13) das Genprodukt des offenen Leserahmens (ORF) yibD von Escherichia coli
überexprimiert.

14. Verfahren gemäss den Ansprüchen 12 und 13, **dadurch gekennzeichnet, dass** man zur Herstellung von L-Threonin Mikroorganismen der Familie Enterobacteriaceae fermentiert, in denen man zusätzlich gleichzeitig eines oder mehrere der Gene, ausgewählt aus der Gruppe:
a) das für die Threonin-Dehydrogenase kodierende tdh-Gen,
b) das für die Malat-Dehydrogenase kodierende mdh-Gen,
c) das Genprodukt des offenen Leserahmens (ORF) yjfA von Escherichia coli,
d) das Genprodukt des offenen Leserahmens (ORF) ytfP von Escherichia coli,
e) das für die Phosphoenolpyruvat-Carboxykinase kodierende pckA-Gen,
f) das für die Pyruvat-Oxidase kodierende poxB-Gen,
g) das für den DgsA-Regulator des Phosphotransferase-Systems kodierende dgsA-Gen,
h) das für den Fructose-Repressor kodierende fruR-Gen,
i) das für den Sigma³⁸-Faktor kodierende rpoS-Gen und,
j) das für die Aspartat Ammonium-Lyase kodierende aspA-Gen
ausschaltet

15. Verfahren gemäss Anspruch 12, **dadurch gekennzeichnet, dass** man L-Aminosäuren, ausgewählt aus der Gruppe L-Asparagin, L-Serin, L-Glutamat, L-Glycin, L-Alanin, L-Cystein, L-Valin, L-Methionin, L-Prolin, L-Isoleucin, L-Leucin, L-Tyrosin, L-Phenylalanin, L-Histidin, L-Lysin, L-Tryptophan, L-Arginin und L-Homoserin herstellt.

16. Verfahren gemäss Anspruch 12, **dadurch gekennzeichnet, dass** man L-Aminosäuren, ausgewählt aus der Gruppe L-Isoleucin, L-Valin, L-Methionin, L-Homoserin, L-Tryptophan, L-Threonin und L-Lysin herstellt.

## Claims

1. Recombinant microorganisms of the family Enterobacteriaceae which contain an overexpressed lamB gene which codes for a polypeptide having the activity of the maltoporin LamB and which produce L-amino acids, and accumulate in the medium, the overexpression being obtained by transforming an L-amino acid-producing strain with a plasmid that carries the lamB gene.

2. Microorganisms according to Claim 1, which additionally contain at least one gene of the thrABC operon coding for aspartate kinase, homoserine dehydrogenase, homoserine kinase and threonine synthase, and produce L-threonine.

3. Microorganisms of the family Enterobacteriaceae according to Claims 1 and 2 in which a polynucleotide is overexpressed which codes for a polypeptide which is up to at least 90%, in particular up to at least 95%, preferably 99%, identical to an amino acid sequence selected from the group SEQ ID No. 4, SEQ ID No. 6 and SEQ ID No. 8, the polypeptide having the activity of the maltoporin LamB.

4. Microorganisms according to Claim 3 containing an overexpressed polynucleotide corresponding to the lamB gene selected from the group:
a) polynucleotide having the nucleotide sequence of SEQ ID No. 3, SEQ ID No. 5 or SEQ ID No. 7;
b) polynucleotide having a nucleotide sequence which corresponds to that of SEQ ID No. 3, SEQ ID No. 5 or SEQ ID No. 7 within the bounds of the degeneracy of the genetic code;
c) polynucleotide having a sequence which hybridizes under stringent conditions with the sequence complementary to the sequence SEQ ID No. 3, SEQ ID No. 5 or SEQ ID No. 7;
d) polynucleotide having a sequence SEQ ID No. 3, SEQ ID No. 5 or SEQ ID No. 7 which contains functionally neutral sense mutants.

5. Microorganisms according to Claim 3, the polypeptide having an amino acid sequence which is at least 95% identical with one of the sequences selected from the group SEQ ID No. 4, SEQ ID No. 6 and SEQ ID No. 8.

6. Microorganisms according to Claim 3, the polypeptide having the amino acid sequence which is identical with that of SEQ ID No. 4, SEQ ID No. 6 or SEQ ID No. 8.

7. Microorganisms according to Claims 1 to 6, in which the number of copies of the lamB gene or of the polynucleotide sequences having this function is present increased by at least 1.

8. Microorganisms according to Claim 7, the increase in number of copies of the lamB gene by at least 1 being effected by integration into the chromosome of the microorganism of the gene or the polynucleotide sequences having this function.

9. Microorganisms according to Claim 7, the increase in the number of copies of the lamB gene by at least 1 being achieved by a vector replicating extrachromosomally.

10. Microorganisms according to Claims 1 to 9, wherein, by amplifying the lamB gene, the concentration or activity of the LamB gene product (protein) is increased by at least 10%, based on the activity or concentration of the gene product in the non-recombinant starting strain.

11. Microorganisms according to Claims 1 to 10, **characterized in that** the microorganisms are selected from the genera Escherichia, Erwinia, Providencia and Serratia.

12. Method for the production of L-amino acids by fermentation of recombinant microorganisms of the family Enterobacteriaceae, **characterized in that**
a) the microorganisms producing the desired L-amino acid according to Claims 1 to 11, are cultured in a medium under conditions in which the desired L-amino acid is enriched in the medium, and
b) the desired L-amino acid is isolated, components of the fermentation broth and/or the biomass in its totality or fractions (≥ 0 to 100%) remaining in the isolated product or being completely removed.

13. Method according to Claim 12, **characterized in that**, to produce L-threonine, microorganisms of the family Enterobacteriaceae are fermented in which, in addition simultaneously one or more of the genes selected from the group:
a) at least one gene of the thrABC operon coding for aspartate kinase, homoserine dehydrogenase, homoserine kinase and threonine synthase,
b) the pyc gene of Corynebacterium glutamicum coding for pyruvate carboxylase,
c) the pps gene coding for phosphoenolpyruvate synthase,
d) the ppc gene coding for phosphoenolpyruvate carboxylase,
e) the genes pntA and pntB coding for the subunits of pyridine transhydrogenase,
f) the gene rhtB coding for the protein conferring homoserine resistance,
g) the gene rhtC coding for the protein conferring threonine resistance,
h) the thrE gene from Corynebacterium glutamicum coding for the threonine-export-carrier protein,
i) the gdhA gene coding for glutamate dehydrogenase,
k) the pgm gene coding for phosphoglucomutase,
l) the fba gene coding for fructose biphosphate aldolase,
m) the ptsH gene coding for phosphohistidine protein hexose phosphotransferase,
n) the ptsI gene coding for enzyme I of the phosphotransferase system,
o) the crr gene coding for the glucose-specific IIA component,
p) the ptsG gene coding for the glucose-specific IIBC component,
q) the lrp gene coding for the regulator of the leucine regulon,
r) the fadR gene coding for the regulator of the fad regulon,
s) the iclR gene coding for the regulator of central intermediate metabolism,
t) the ahpC gene coding for the small subunit of alkyl hydroperoxide reductase,
u) the ahpF gene coding for the large subunit of alkyl hydroperoxide reductase,
v) the cysK gene coding for cysteine synthase A,
w) the cysB gene coding for the regulator of the cys regulon,
x) the cysJ gene coding for the flavoprotein of NADPH sulfite reductase,
y) the cysI gene coding for the hemoprotein of NADPH sulfite reductase,
z) the cysH gene coding for adenylylsulfate reductase,
z1) the rseA gene coding for a membrane protein having anti-sigmaE activity,
z2) the rseC gene coding for a global regulator of the sigmaE factor,
z3) the sucA gene coding for the decarboxylase subunit of 2-ketoglutarate dehydrogenase,
z4) the sucB gene coding for the dihydrolipoyltranssuccinase E2 subunit of 2-ketoglutarate dehydrogenase,
z5) the sucC gene coding for the β-subunit of succinyl-CoA synthetase,
z6) the sucD gene coding for the α-subunit of succinyl-CoA synthetase,
z7) the aceE gene coding for the E1 component of the pyruvate dehydrogenase complex,
z8) the aceF gene coding for the E2 component of the pyruvate dehydrogenase complex,
z9) the rseB gene coding for the regulator of SigmaE factor activity,
z10) the malT gene coding for the positive transcriptional regulator of the maltose regulon,
z11) the gene product of the open reading frame (ORF) yaaU of Escherichia coli,
z12) the gene product of the open reading frame (ORF) yodA of Escherichia coli and
z13) the gene product of the open reading frame (ORF) yibD of Escherichia coli
is or are overexpressed.

14. Method according to Claims 12 and 13, **characterized in that** to produce L-threonine, microorganisms of the family Enterobacteriaceae are fermented in which, in addition simultaneously one or more of the genes selected from the group:
a) the tdh gene coding for threonine dehydrogenase,
b) the mdh gene coding for malate dehydrogenase,
c) the gene product of the open reading frame (ORF) yjfA of Escherichia coli,
d) the gene product of the open reading frame (ORF) ytfP of Escherichia coli,
e) the pckA gene coding for phosphoenolpyruvate carboxykinase,
f) the poxB gene coding for pyruvate oxidase,
g) the dgsA gene coding for the DgsA regulator of the phosphotransferase system,
h) the fruR gene coding for the fructose repressor,
i) the rpoS gene coding for the Sigma³⁸ factor,
j) the aspA gene coding for aspartate ammonium lyase
is or are turned off.

15. Method according to Claim 12, **characterized in that** L-amino acids selected from the group L-asparagine, L-serine, L-glutamate, L-glycine, L-alanine, L-cysteine, L-valine, L-methionine, L-proline, L-isoleucine, L-leucine, L-tyrosine, L-phenylalanine, L-histidine, L-lysine, L-tryptophan, L-arginine and L-homoserine are produced.

16. Method according to Claim 12, **characterized in that** L-amino acids selected from the group L-isoleucine, L-valine, L-methionine, L-homoserine, L-tryptophan, L-threonine and L-lysine are produced.

## Revendications

1. Micro-organismes recombinants de la famille des *Enterobacteriaceae,* qui contiennent un gène lamB surexprimé, qui code pour un polypeptide ayant l'activité de la maltoporine LamB, et qui produisent des L-aminoacides, et s'accumulent dans le milieu, la surexpression étant obtenue par la transformation d'une souche productrice de L-aminoacides par un plasmide qui porte le gène lamB.

2. Micro-organismes selon la revendication 1, qui contiennent en outre au moins un gène de l'opéron thrABC codant pour l'aspartate kinase, l'homosérine déshydrogénase, l'homosérine kinase et la thréonine synthase et produisent de la L-thréonine.

3. Micro-organismes de la famille des *Enterobacteriaceae* selon les revendications 1 et 2, dans lesquels est surexprimé un polynucléotide qui code pour un polypeptide qui est identique à raison d'au moins 90 %, en particulier à raison d'au moins 95 %, de préférence 99 % à une séquence d'aminoacides choisie dans le groupe constitué par SEQ ID n° 4, SEQ ID n° 6 et SEQ ID n° 8, le polypeptide possédant l'activité de la maltoporine LamB.

4. Micro-organismes selon la revendication 3, contenant un polynucléotide surexprimé correspondant au gène lamB, choisi dans le groupe:
a) polynucléotide ayant la séquence nucléotidique de SEQ ID n° 3, SEQ ID n° 5 ou SEQ ID n° 7 ;
b) polynucléotide ayant une séquence nucléotidique qui correspond à la SEQ ID n° 3, SEQ ID n° 5 ou SEQ ID n° 7 dans le cadre de la dégénérescence du code génétique ;
c) polynucléotide ayant une séquence qui dans des conditions stringentes s'hybride avec la séquence complémentaire de la séquence SEQ ID n° 3, SEQ ID n° 5 ou SEQ ID n° 7 ;
d) polynucléotide ayant une séquence SEQ ID n° 3, SEQ ID n° 5 ou SEQ ID n° 7, qui contient des mutants sens neutres quant à la fonction.

5. Micro-organismes selon la revendication 3, dans lesquels le polypeptide possède une séquence d'aminoacides qui est identique à raison d'au moins 95 % à l'une des séquences choisie dans le groupe constitué par SEQ ID n° 4, SEQ ID n° 6 et SEQ ID n° 8.

6. Micro-organismes selon la revendication 3, dans lesquels le polypeptide présente une séquence d'aminoacides qui est identique à celle de SEQ ID n° 4, SEQ ID n° 6 ou SEQ ID n° 8.

7. Micro-organismes selon les revendications 1 à 6, dans lesquels le nombre de copies du gène lamB ou des séquences de polynucléotides ayant cette fonction est augmenté d'au moins 1.

8. Micro-organismes selon la revendication 7, dans lesquels l'augmentation d'au moins 1 du nombre de copies du gène lamB est réalisée par intégration du gène ou des séquences de polynucléotides ayant cette fonction, dans le chromosome des micro-organismes.

9. Micro-organismes selon la revendication 7, dans lesquels l'augmentation d'au moins 1 du nombre de copies du gène lamB est atteinte au moyen d'un vecteur à réplication extrachromosomique.

10. Micro-organismes selon les revendications 1 à 9, dans lesquels la concentration ou l'activité du produit (de la protéine) du gène lamB est augmentée d'au moins 10 % par le renforcement du gène lamB, par rapport à l'activité ou à la concentration du produit du gène chez la souche non recombinante de départ.

11. Micro-organismes selon les revendications 1 à 10, **caractérisés en ce que** les micro-organismes sont choisis dans les genres *Escherichia, Erwinia,* Providence et *Serratia.*

12. Procédé pour la production de L-aminoacides par culture par fermentation de micro-organismes recombinants de la famille des *Enterobacteriaceae,* **caractérisé en ce que**
a) on cultive dans un milieu les micro-organismes selon les revendications 1 à 11, producteurs du L-aminoacide désiré, dans des conditions dans lesquelles le L-aminoacide désiré est accumulé dans le milieu, et
b) on isole le L-aminoacide désiré, des composants du bouillon de fermentation et/ou la biomasse restant en leur totalité ou en parties (≥ 0 à 100 %) dans le produit isolé ou étant totalement éliminés.

13. Procédé selon la revendication 12, **caractérisé en ce que** pour la production de L-thréonine on cultive par fermentation des micro-organismes de la famille des *Enterobacteriaceae,* dans lesquels en outre on surexprime en même temps un ou plusieurs des gènes choisis dans le groupe constitué par:
a) au moins un gène de l'opéron thrABC codant pour l'aspartate kinase, l'homosérine déshydrogénase, l'homosérine kinase et la thréonine synthase,
b) le gène pyc de *Corynebacterium glutamicum*, codant pour la pyruvate carboxylase,
c) le gène pps codant pour la phosphoénolpyruvate synthase,
d) le gène ppc codant pour la phosphoénolpyruvate carboxylase,
e) les gènes pntA et pntB codant pour les sous-unités de la pyridine transhydrogénase,
f) le gène rhtB codant pour la protéine conférant la résistance à l'homosérine,
g) le gène rhtC codant pour la protéine conférant la résistance à la thréonine,
h) le gène thrE de *Corynebacterium glutamicum* codant pour la protéine export-carrier de thréonine,
i) le gène gdhA codant pour la glutamate déshydrogénase,
k) le gène pgm codant pour la phosphoglucomutase,
1) le gène fba codant pour la fructose biphosphate aldolase,
m) le gène ptsH codant pour la phosphohistidine- protéine-hexose phosphotransférase,
n) le gène ptsI codant pour l'enzyme I du système phosphotransférase,
o) le gène crr codant pour le composant IIA spécifique du glucose,
p) le gène ptsG codant pour le composant IIBC spécifique du glucose,
q) le gène lrp codant pour le régulateur du régulon leucine,
r) le gène fadR codant pour le régulateur du régulon fad,
s) le gène iclR codant pour le régulateur du métabolisme intermédiaire central,
t) le gène ahpC codant pour la petite sous-unité de l'alkyle hydroperoxyde réductase,
u) le gène ahpF codant pour la grande sous-unité de l'alkyle hydroperoxyde réductase,
v) le gène cysK codant pour la cystéine synthase A,
w) le gène cysB codant pour le régulateur du régulon cys,
x) le gène cysJ codant pour la flavoprotéine de la NADPH-sulfite réductase,
y) le gène cysI codant pour l'hémoprotéine de la NADPH-sulfite réductase,
z) le gène cysH codant pour l'adénylylsulfate réductase,
z1) le gène rseA codant pour une protéine membranaire à activité anti-sigmaE,
z2) le gène rseC codant pour un régulateur global du facteur sigmaE,
z3) le gène sucA codant pour la sous-unité décarboxylase de la 2-cétoglutarate déshydrogénase,
z4) le gène sucB codant pour la sous-unité dihydro- lipoyltrans-succinase E2 de la 2-cétoglutarate déshydrogénase,
z5) le gène sucC codant pour la sous-unité β de la succinyl-CoA synthétase,
z6) le gène sucD codant pour la sous-unité α de la succinyl-CoA synthétase,
z7) le gène aceE codant pour le composant E1 du complexe pyruvate-déshydrogénase,
z8) le gène aceF codant pour le composant E2 du complexe pyruvate-déshydrogénase,
z9) le gène rseB codant pour le régulateur de l'activité du facteur SigmaE,
z10) le gène malT codant pour le régulateur transcriptionnel positif du régulon maltose,
z11) le produit génique du cadre de lecture ouvert (ORF) yaaU d'*Escherichia coli,*
z12) le produit génique du cadre de lecture ouvert (ORF) yodA d'*Escherichia coli,*
z13) le produit génique du cadre de lecture ouvert (ORF) yibD d'*Escherichia coli.*

14. Procédé selon les revendications 12 et 13, **caractérisé en ce que** pour la production de L-thréonine on cultive par fermentation des micro-organismes de la famille des *Enterobacteriaceae,* dans lesquels en outre on inactive en même temps un ou plusieurs des gènes choisis dans le groupe constitué par :
a) le gène tdh codant pour la thréonine déshydrogénase,
b) le gène mdh codant pour la malate déshydrogénase,
c) le produit génique du cadre de lecture ouvert (ORF) yj fA d'*Escherichia coli,*
d) le produit génique du cadre de lecture ouvert (ORF) ytfP d'*Escherichia coli,*
e) le gène pckA codant pour la phosphoénolpyruvate carboxykinase,
f) le gène poxB codant pour la pyruvate oxydase,
g) le gène dgsA codant pour le régulateur DgsA du système phosphotransférase,
h) le gène fruR codant pour le répresseur de fructose,
i) le gène rpoS codant pour le facteur Sigma³⁸,
j) le gène aspA codant pour l'aspartate ammonium lyase.

15. Procédé selon la revendication 12, **caractérisé en ce qu'**on produit des L-aminoacides, choisis dans le groupe constitué par la L-asparagine, la L-sérine, le L-glutamate, la L-glycine, la L-alanine, la L-cystéine, la L-valine, la L-méthionine, la L-proline, la L-isoleucine, la L-leucine, la L-tyrosine, la L-phénylalanine, la L-histidine, la L-lysine, le L-tryptophane, la L-arginine et la L-homosérine.

16. Procédé selon la revendication 12, **caractérisé en ce qu'**on produit des L-aminoacides, choisis dans le groupe constitué par la L-isoleucine, la L-valine, la L-méthionine, la L-homosérine, le L-tryptophane, la L-thréonine et la L-lysine.
